# EUROPEAN PATENT SPECIFICATION

(11) **EP 0 584 085 B1**
(45) Date of publication and mention of the grant of the patent: **09.09.1998**
(21) Application number: 92907243.7
(22) Date of filing: 02.04.1992
(51) Int. Cl.: C07D 477/00, C07D 207/16, C07F 9/568, C07D 403/12, A61K 31/40

(54) **ANTIBIOTIC CARBAPENEM DERIVATIVES**
ANTIBIOTISCHE CARBAPENEMDERIVATE
DERIVES ANTIBIOTIQUES DE CARBAPENEM

(30) Priority: 08.04.1991 GB 9107363
(43) Date of publication of application: 02.03.1994
(73) Proprietor: ZENECA LIMITED, London W1Y 6LN (GB); ZENECA Pharma S.A., 95022 Cergy Pontoise Cédex (FR)
(72) Inventor: BETTS, Michael, John, Cheshire SK9 6LB (GB); BREAULT, Gloria, Anne, Cheshire CW12 3RA (GB)
(74) Representative: Denerley, Paul Millington, Dr.
(86) International application number: GB9200586
(87) International publication number: WO9217479

(56) References cited:
- EP-A- 0 126 587
- EP-A- 0 182 213
- EP-A- 0 243 686
- EP-A- 0 443 883
- EP-A- 0 472 062

## Description

The present invention relates to carbapenems and in particular to such compounds containing a dihydroxybenzene or related group. This invention further relates to processes for their preparation, to intermediates in their preparation, to their use as therapeutic agents and to pharmaceutical compositions containing them. The compounds of this invention are antibiotics and can be used in the treatment of any disease that is conventionally treated with antibiotics for example in the treatment of bacterial infection in mammals including humans.

Carbapenems were first isolated from fermentation media in 1974 and were found to have broad spectrum antibacterial activity. Since this discovery substantial investigations have been made into new carbapenem derivatives and many hundreds of patents and scientific papers have been published.

The first, and so far the only, carbapenem to be commercially marketed is imipenem (N-formimidoyl thienamycin). This compound has a broad spectrum of antibacterial activity.

The present invention provides compounds with particularly good antibacterial activity in particular against strains of Pseudomonas aeruginosa and those strains of this organism which have been shown to be resistant to imipenem. They exhibit good stability to beta-lactamases. In addition many of the compounds of this invention exhibit favourable pharmacokinetics.

The carbapenem derivatives referred to herein are named in accordance with the generally accepted semi-systematic nomenclature:

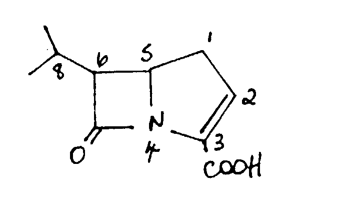

Accordingly the present invention provides a compound of the formula (I) 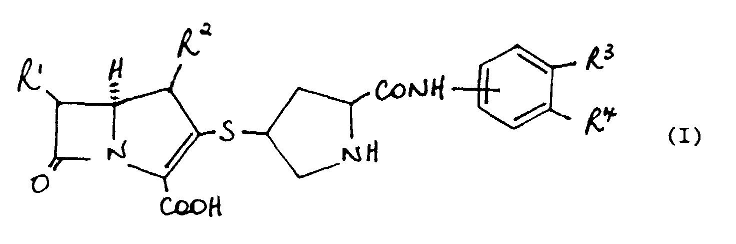 wherein:
R¹ is 1-hydroxyethyl, 1-fluoroethyl or hydroxymethyl;
R² is hydrogen or C₁₋₄alkyl;
R³ and R⁴ are ortho with respect to one another wherein R³ and R⁴ are independently hydroxy or in vivo hydrolysable esters thereof; the benzene ring being optionally further substituted;
or a pharmaceutically acceptable salt or in vivo hydrolysable precursor thereof.

Preferably R¹ is 1-hydroxyethyl.
R² is hydrogen or C₁₋₄alkyl for example methyl, ethyl, n-propyl, isopropyl and n-butyl. Preferably R² is hydrogen or methyl and in particular R² is methyl.
R³ is hydroxy or an in vivo hydrolysable ester thereof. R⁴ is hydroxy or an in vivo hydrolysable ester thereof.

In vivo hydrolysable esters are those pharmacetuically acceptable esters that hydrolyse in the human body to produce the parent hydroxy or carboxy compound. Such esters can be identified by administering, eg. intravenously to a test animal, the compound under test and subsequently examining the test animal's body fluids. Suitable in vivo hydrolysable esters for hydroxy include acetoxy, propionyloxy, pivaloyloxy, C₁₋₄alkoxycarbonyloxy for example ethoxycarbonyloxy, phenylacetoxy and phthalidyl. Suitable in vivo hydrolysable esters for carboxy include C₁₋₆alkoxymethyl esters for example methoxymethyl; C₁₋₆alkanoyloxymethyl esters for example pivaloyloxymethyl; C₃₋₈cycloalkoxycarbonyloxyC₁₋₆alkyl, for example 1-cyclohexyloxycarbonyloxyethyl; 1,3-dioxolen-2-onylmethyl esters for example 5-methyl-1,3-dioxolen-2-onylmethyl; phthalidyl esters and C₁₋₆alkoxycarbonyloxyethyl esters for example 1-ethoxycarbonyloxyethyl and may be formed at any carboxy group in the compound of this invention.

Conveniently R³ and R⁴ have the same value and are both hydroxy or are both in vivo hydrolysable esters, for example they are both acetoxy or pivaloyloxy.

As stated hereinbefore the benzene ring is optionally further substituted. Particular optional substituents include C₁₋₄alkyl for example methyl, ethyl or isopropyl; halo for example chloro, bromo or fluoro; hydroxy; hydroxyC₁₋₄alkyl for example hydroxymethyl or hydroxyethyl; amino; nitro; C₁₋₄alkoxy for example methoxy or ethoxy; carboxyC₁₋₄alkyl for example carboxymethyl or carboxyethyl; C₁₋₄alkanoylamino for example acetamido; N-alkyl-N-C₁₋₄alkanoylamino; trifluoromethyl; carboxy; carbamoyl; C₁₋₄alkylcarbamoyl for example methylcarbamoyl; di-C₁₋₄alkylcarbamoyl for example di-[N-ethyl]carbamoyl; cyano; C₁₋₄alkanesulphonamido for example methanesulphonamido; C₁₋₄alkanoyl for example acetyl; C₁₋₄alkanoyloxy for example acetoxy or propionoxy; C₁₋₄alkoxycarbonyl for example methoxycarbonyl; C₁₋₄alkylthio for example methylthio, C₁₋₄alkanesulphinyl for example methanesulphinyl; C₁₋₄alkanesulphonyl for example methanesulphonyl; C₂₋₄alkenyl for example allyl or vinyl; hydroxyiminomethyl (HON=CH-); C₁₋₄alkoxyiminomethyl for example methoxyiminomethyl; aminosulphonyl; N-C₁₋₄alkylaminosulphonyl for example N-methylaminosulphonyl; and di-[N-C₁₋₄alkyl]aminosulphonyl for example di-[N-methyl]aminosulphonyl.

Favoured optional substituents for the benzene ring are fluoro, bromo, chloro, cyano, nitro, carboxymethyl, hydroxy, di-[N-methyl]carbamoyl, methanesulphonyl, di-[N-ethyl]aminosulphonyl and methoxycarbonyl.

The skilled man will realise that the benzene ring may have up to 3 optional substituents, which may be the same or different, are possible. In general we prefer up to 2 optional substituents.

The present invention covers all epimeric, diastereoisomeric and tautomeric forms of the compounds of the formula (I) except where otherwise indicated. The compounds of the formula (I) have a number of centres of optical activity, namely: within the group R¹ (when R¹ is 1-hydroxyethyl or 1-fluoroethyl); at the 6-position; at the 1-position (when R² is C₁₋₄alkyl); and at the 2' and 4' positions in the pyrrolidine ring:

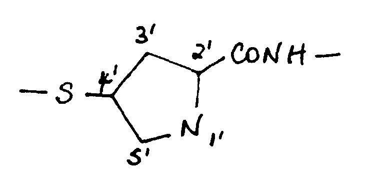

Preferred compounds are those in which the beta-lactam protons are in trans configuration with respect to one another. When R¹ is 1-hydroxyethyl or 1-fluoroethyl it is preferred that the 8-substituent has the R-configuration. Thus a preferred class of compounds is that of the formula (III): 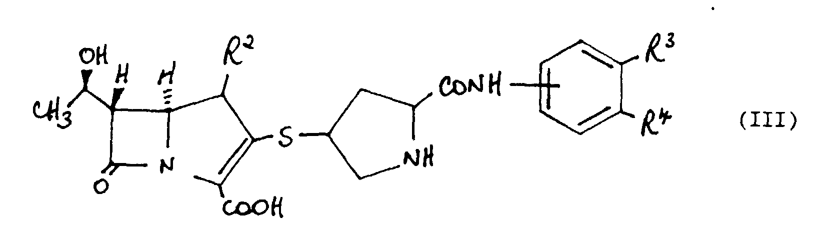 and pharmaceutically acceptable salts and in vivo hydrolysable esters thereof, wherein R², R³ and R⁴ are as hereinbefore defined and the benzene ring is further optionally substituted.

In the formulae herein, when a bond is represented as a wedge, this idicates that in three dimensions the bond would be coming forward out of the paper and when a bond is represented as hatched this bond would be going back into the paper.

When R² is C₁₋₄alkyl for example methyl it is preferred that the compound is in the form of the 1R configuration.

A particularly preferred class of compounds of the present invention is that of the formula (IV): 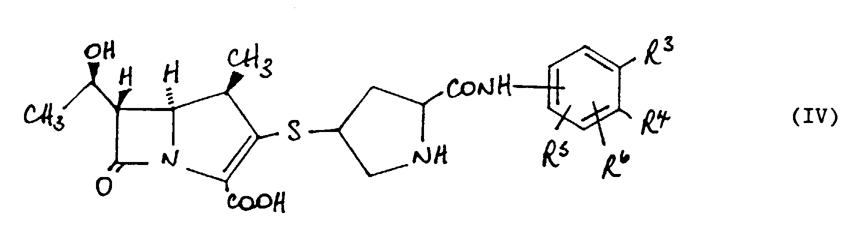 wherein R³ and R⁴ are hereinbefore defined and R⁵ and R⁶ are independently hydrogen, halo, cyano, nitro, carboxy, carboxymethyl, methanesulphonyl, di-[N-methyl]carbamoyl, di-[N-ethyl]aminosulphonyl and hydroxy. Preferably the benzene ring is substituted by R³ and R⁴ in positions 3 and 4 relative to the amido linking group. Particular substitution patterns for the benzene ring include: 3,4-dihydroxybenzene, 3,4-dihydroxy-5-bromo-benzene, 2,5-dichloro-3,4-dihydroxybenzene, 3,4,5-trihydroxybenzene, 3,4-dihydroxy-6-methoxycarbonylbenzene, 3,4-dihydroxy-5-cyanobenzene and 3,4-dihydroxy-6-di-[N-ethyl]aminosulphonylbenzene.

Particular compounds of the present invention are
(5R,6S,8R,2'S,4'S)-2-(2-(3,4-dihydroxyphenylcarbamoyl)pyrrolidin-4-ylthio)-6-(1-hydroxyethyl)carbapenem-3-carboxylic acid;
(1R,5R,6S,8R,2'S,4'S)-2-(2-(5-cyano-3,4-dihydroxyphenylcarbamoyl)pyrrolidin-4-ylthio)-6-(1-hydroxyethyl)-1-methylcarbapenem-3-carboxylic acid;
(5R,6S,8R,2'S,4'S)-2-(2-(5-bromo-3,4-dihydroxyphenylcarbamoyl)pyrrolidin-4-ylthio)-6-(1-hydroxyethyl)carbapenem-3-carboxylic acid;
(1R,5R,6S,8R,2'S,4'S)-2-(2-(3,4-dihydroxyphenylcarbamoyl)pyrrolidin-4-ylthio)-6-(1-hydroxyethyl)-1-methylcarbapenem-3-carboxylic acid;
(1R,5R,6S,8R,2'S,4'S)-2-(2-(5-methoxy-3,4-dihydroxyphenylcarbamoyl)pyrrolidin-4-ylthio)-6-(1-hydroxyethyl)-1-methylcarbapenem-3-carboxylic acid;
(1R,5R,6S,8R,2'S,4'S)-2-(2-(2,5-dichloro-3,4-dihydroxyphenyl-carbamoyl)pyrrolidin-4-ylthio)-6-(1-hydroxyethyl)-1-methylcarbapenem-3-carboxylic acid;
(1R,5R,6S,8R,2'S,4'S)-2-(2-(5-hydroxy-3,4-dihydroxyphenylcarbamoyl)pyrrolidin-4-ylthio)-6-(1-hydroxyethyl)-1-methylcarbapenem-3-carboxylic acid;
(1R,5R,6S,8R,2'S,4'S)-2-(2-(5-bromo-3,4-dihydroxyphenylcarbamoyl)pyrrolidin-4-ylthio)-6-(1-hydroxyethyl)-1-methylcarbapenem-3-carboxylic acid;
(1R,5R,6S,8R,2'S,4'S)-2-(2-(5-N-methoxyiminomethyl-3,4-dihydroxyphenylcarbamoyl)pyrrolidin-4-ylthio)-6-(1-hydroxyethyl)-1-methylcarbapenem-3-carboxylic acid;
(1R,5R,6S,8R,2'S,4'S)-2-(2-(6-methoxycarbonyl-3,4-dihydroxyphenylcarbamoyl)pyrrolidin-4-ylthio)-6-(1-hydroxyethyl)-1-methylcarbapenem-3-carboxylic acid;
(1R,5R,6S,8R,2'S,4'S)-2-(2-(6-carboxy-3,4-dihydroxyphenylcarbamoyl)pyrrolidin-4-ylthio)-6-(1-hydroxyethyl)-1-methylcarbapenem-3-carboxylic acid; and
(1R,5R,6S,8R,2'S,4'S)-2-(2-(6-di-(N-ethyl)aminosulphonyl-3,4-dihydroxyphenylcarbamoyl)pyrrolidin-4-ylthio)-6-(1-hydroxyethyl)-1-methylcarbapenem-3-carboxylic acid;

Suitable salts include acid addition salts such as hydrochloride, hydrobromide, citrate, maleate and salts formed with phosphoric and sulphuric acid. In another aspect suitable salts are base salts such as an alkali metal salt for example sodium or potassium, an alkaline earth metal salt for example calcium or magnesium, an organic amine salt for example triethylamine, morpholine, N-methylpiperidine, N-ethylpiperidine, procaine, dibenzylamine, or N,N-dibenzylethylamine, or amino acids, for example, lysine.

For the avoidance of doubt there may be two or three salt forming counter ions depending on the number and type of charged functions and the valency of the counter ions.

In order to use a compound of the present invention or a pharmaceutically acceptable salt or in vivo hydrolysable precursor thereof for the therapeutic treatment of mammals including humans, in particular in treating infection, it is normally formulated in accordance with standard pharmaceutical practice as a pharmaceutical composition.

Therefore in another aspect the present invention provides a pharmaceutical composition which comprises a compound of the formula (I) or a pharmaceutically acceptable salt or in vivo hydrolysable precursor thereof and a pharmaceutically acceptable carrier.

The pharmaceutical compositions of this invention may be administered in standard manner for the disease condition that it is desired to treat, for example by oral, rectal or parenteral administration. For these purposes the compounds of this invention may be formulated by means known in the art into the form of, for example, tablets, capsules, aqueous or oily solutions or suspensions, emulsions, dispersible powders, suppositories and sterile injectable aqueous or oily solutions or suspensions.

In addition to the compounds of the present invention the pharmaceutical composition of this invention may also contain, or be co-administered with, one or more known drugs selected from other clinically useful antibacterial agents (for example other beta-lactams or aminoglycosides), inhibitors of beta-lactamase (for example clavulanic acid), renal tubular blocking agents (e.g. probenecid), inhibitors of metabolising enzymes (for example inhibitors of peptidases, for example Z-2-acylamino-3-substituted propenoates such as cilastatin) and N-acylated amino acids (for example see EP-A-178911).

A suitable pharmaceutical composition of this invention is one suitable for oral administration in unit dosage form, for example a tablet or capsule which contains between 100mg and 1g of the compound of this invention.

A preferred pharmaceutical composition of the invention is one suitable for intravenous, subcutaneous or intramuscular injection, for example a sterile injectable containing between 1 and 50% w/w of the compound of this invention.

The pharmaceutical compositions of the invention will normally be administered to man in order to combat infections caused by bacteria, in the same general manner as that employed for imipenem due allowance being made in terms of dose levels for the potency of the compound of the present invention relative to the clinical use of imipenem. Thus each patient will receive a daily intravenous, subcutaneous or intramuscular dose of 0.05 to 5g., and preferably 0.1 to 2.5g., of the compound of this invention, the composition being administered 1 to 4 times per day, preferably 1 or 2 times a day. The intravenous, subcutaneous and intramuscular dose may be given by means of a bolus injection. Alternatively the intravenous dose may be given by continuous infusion over a period of time. Alternatively each patient will receive a daily oral dose which is approximately equivalent to the daily parenteral dose. Thus a suitable daily oral dose is 0.5 to 5g. of the compound of this invention, the composition being administered 1 to 4 times per day.

In a further aspect the present invention provides a process for preparing the compounds of the formula (I) or a pharmaceutically acceptable salt or in vivo hydrolysable ester thereof which process comprises deprotecting a compound of the formula (V): 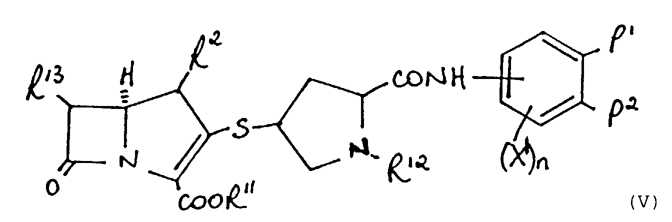 wherein R² is as hereinbefore defined; R¹³ is a group R¹ or 1-(protected hydroxy)ethyl; R¹¹ is hydrogen or a carboxy protecting group; R¹² is hydrogen or an amino protecting group, P¹ and P² are independently hydroxy or protected hydroxy and (X¹)ₙ represents up to three optional substituents which may be the same or different selected from C₁₋₄alkyl, halo, hydroxy, hydroxyC₁₋₄alkyl, amino, nitro, C₁₋₄alkoxy, carboxyC₁₋₄alkyl, C₁₋₄alkanoylamino, N-alkyl-N-C₁₋₄alkanoylamino, trifluoromethyl, carboxy, carbamoyl, C₁₋₄alkylcarbamoyl, di-C₁₋₄alkylcarbamoyl, cyano, C₁₋₄alkanesulphonamido, C₁₋₄alkanoyl, C₁₋₄alkanoyloxy, C₁₋₄alkoxycarbonyl, C₁₋₄alkylthio, C₁₋₄alkanesulphinyl, C₁₋₄alkanesulphonyl, C₂₋₄alkenyl, hydroxyiminomethyl (HON=CH-), C₁₋₄alkoxyiminomethyl, aminosulphonyl, N-C₁₋₄alkylaminosulphonyl, and di-[N-C₁₋₄alkyl]aminosulphonyl; with the proviso that there is at least one protecting group:
and thereafter if necessary:
(i) forming a pharmaceutically acceptable salt,
(ii) esterifying to form an in vivo hydrolysable ester.

The compounds of the formula (V) are novel and form another aspect of the invention.

Protecting groups may in general be chosen from any of the groups described in the literature or known to the skilled chemist as appropriate for the protection of the group in question, and may be introduced by conventional methods.

Protecting groups may be removed by any convenient method as described in the literature or known to the skilled chemist as appropriate for the removal of the protecting group in question, such methods being chosen so as to effect removal of the protecting group with minimum disturbance of groups elsewhere in the molecule.

Specific examples of protecting groups are given below for the sake of convenience, in which "lower" signifies that the group to which it is applied preferably has 1-4 carbon atoms. It will be understood that these examples are not exhaustive. Where specific examples of methods for the removal of protecting groups are given below these are similarly not exhaustive. The use of protecting groups and methods of deprotection not specifically mentioned is of course within the scope of the invention.

A carboxy protecting group may be the residue of an ester-forming aliphatic or araliphatic alcohol or of an ester-forming silanol (the said alcohol or silanol preferably containing 1-20 carbon atoms).

Examples of carboxy protecting groups include straight or branched chain (1-12C)alkyl groups (eg isopropyl, t-butyl); lower alkoxy lower alkyl groups (eg methoxymethyl, ethoxymethyl, isobutoxymethyl); lower aliphatic acyloxy lower alkyl groups, (eg acetoxymethyl, propionyloxymethyl, butyryloxymethyl, pivaloyloxymethyl); lower alkoxycarbonyloxy lower alkyl groups (eg 1-methoxycarbonyloxyethyl, 1-ethoxycarbonyloxyethyl); aryl lower alkyl groups (eg p-methoxybenzyl, o-nitrobenzyl, p-nitrobenzyl, benzhydryl and phthalidyl); tri(lower alkyl)silyl groups (eg trimethylsilyl and t-butyldimethylsilyl); diaryl(lower alkyl)silyl groups (eg t-butyldiphenylsilyl); tri(lower alkyl)silyl lower alkyl groups (eg trimethylsilylethyl); and (2-6C)alkenyl groups (eg allyl and vinylethyl).

Methods particularly appropriate for the removal of carboxyl protecting groups include for example acid-, base-, metal- or enzymically-catalysed hydrolysis, for groups such as p-nitrobenzyloxycarbonyl, hydrogenation and for groups such as o-nitrobenzyloxycarbonyl, photolytically.

Examples of hydroxy protecting groups include lower alkenyl groups (eg allyl); lower alkanoyl groups (eg acetyl); lower alkoxycarbonyl groups (eg t-butoxycarbonyl); lower alkenyloxycarbonyl groups (eg allyloxycarbonyl); aryl lower alkoxycarbonyl groups (eg benzoyloxycarbonyl, p-methoxybenzyloxycarbonyl, o-nitrobenzyloxycarbonyl, p-nitrobenzyloxycarbonyl); tri lower alkylsilyl (eg trimethylsilyl, t-butyldimethylsilyl) and aryl lower alkyl (eg benzyl) groups. In addition two hydroxy groups substituted on adjacent carbon atoms, for example in the catechol moiety, may be protected in the form of a cyclic silyl ether.

Examples of amino protecting groups include formyl, aralkyl groups (eg benzyl and substituted benzyl, eg p-methoxybenzyl, nitrobenzyl and 2,4-dimethoxybenzyl, and triphenylmethyl); di-p-anisylmethyl and furylmethyl groups; lower alkoxycarbonyl (eg t-butoxycarbonyl); lower alkenyloxycarbonyl groups (eg allyloxycarbonyl); aryl lower alkoxycarbonyl groups (eg benzoyloxycarbonyl, p-methoxybenzyloxycarbonyl, o-nitrobenzyloxycarbonyl, p-nitrobenzyloxycarbonyl); trialkylsilyl (eg trimethylsilyl and t-butyldimethylsilyl); alkylidene (eg methylidene); benzylidene and substituted benzylidene groups; and the phthalimido group.

Methods appropriate for removal of hydroxy and amino protecting groups include, for example, acid-. base-, metal-, or enzymically-catalysed hydrolysis, for groups such as p-nitrogenzyloxycarbonyl, hydrogenation and for groups such as o-nitrobenzyloxycarbonyl, photolytically.

In another aspect of the present invention the compounds of the formula (I) or pharmaceutically acceptable salts or in vivo hydrolysable esters thereof and compounds of the formula (V) may be prepared by:
a) reacting compounds of the formulae (VI) and (VII): 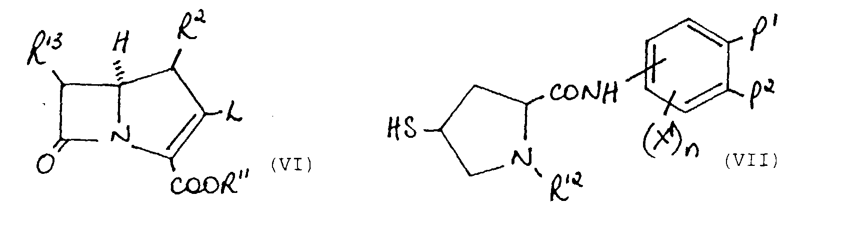 wherein P¹, P², R², X¹, n, R¹¹, R¹² and R¹³ are as hereinbefore defined and L is a leaving group, or
b) cyclising a compound of the formula (VIII): 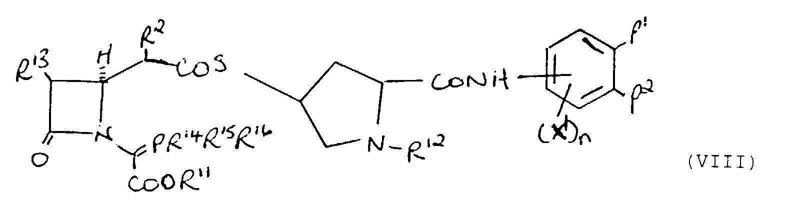 wherein P¹, P², X¹, n, R², R¹¹, R¹² and R¹³ are as hereinbefore defined and R¹⁴, R¹⁵ and R¹⁶ are independently selected from C₁₋₆alkoxy, aryloxy, di-C₁₋₆alkylamino and diarylamino or any two of R¹⁴-R¹⁶ represent o-phenylenedioxy; wherein any functional group is optionally protected and thereinafter if necessary:
   (i) removing any protecting groups;
   (ii) forming a pharmaceutically acceptable salt;
   (iii) esterifying to form an in vivo hydrolysable ester.

Suitably L is the reactive ester of a hydroxy group such as a sulphonate (for example C₁₋₆alkanesulphonyloxy, trifluoromethanesulphonyloxy, benzenesulphonyloxy, toluenesulphonyloxy), a phosphoric ester (for example a diarylphosphoric ester such as diphenylphosphoric ester) or L is a halide (for example chloride). In an alternative L is a sulphoxide for example -SO-CH=CH-NHCOCH₃ which may be readily displaced. Preferably L is diphenylphosphoric ester (-OP(O) (OPh)₂).

Compounds of the formula (VI) and their preparation are well known in the carbapenem literature, for example see EP-A-126587, EP-A-160391, EP-A-243686 and EP-A-343499.

The reaction between the compounds of the formulae (VI) and (VII) is typically performed in the presence of a base such as an organic amine for example di-isopropylethylamine or an inorganic base for example an alkali metal carbonate such as potassium carbonate. The reaction is conveniently performed at a temperature between -25°C and ambient, suitably at about 0°C. The reaction is generally performed in an organic solvent such as acetonitrile or dimethylformamide. The reaction is generally performed in a manner similar to that described in the literature for similar reactions.

The compounds of the formula (VII) are novel and form another aspect of the present invention.

The compounds of the formula (VII) may be prepared by the deprotection of a compound of the formula (IX): 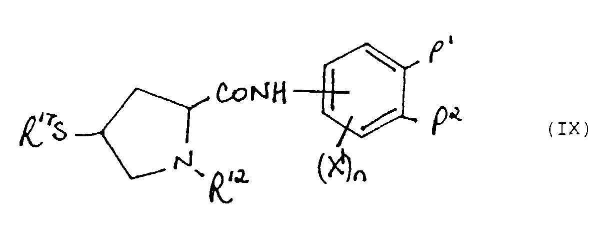 wherein P¹, P², X¹, n and R¹² are as hereinbefore defined and R¹⁷ is a protecting group, for example C₁₋₆alkanoyl or C₁₋₆ alkoxycarbonyl. Preferred values for R¹⁷ are acetyl and t-butoxycarbonyl. The compounds of the formula (IX) can be converted to the compounds of the formula (VII) by standard methods of deprotection, for example acetyl groups can be removed by basic hydrolysis in aqueous alkanol, alkenol for example allyl alcohol or tetrahydrofuran.

The compounds of the formula (IX) are novel and form another aspect of the present invention.

The compounds of the formula (IX) may be prepared by the reaction of a compound of the formula (X) or activated derivative thereof which may be formed in situ with a compound of the formula (XI): 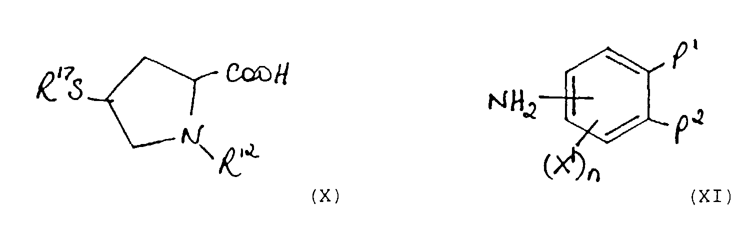 wherein P¹, P², X¹, n, R¹² and R¹⁷ are as hereinbefore defined. Activated derivatives of the compound of the formula (X) include activated esters, anhydrides such as 1H-benzol[1,2,3]triazol-1-yl, pentafluorophenyl and 2,4,5-trichlorophenyl esters or the benzimidazol-2-yl ester of the thiocarboxylic acid corresponding to (X) and acid halides. The reaction of the compounds of the formulae (X) and (XI) is performed under standard methods of acylating anilines and related compounds, for example in the presence of Vilsmeier reagent (thus forming the reactive derivative of (X) in situ) at temperatures in the range of -30°C to +25°C, preferably in the region -20°C to +5°C, or in the presence of sulphonyl chloride at ambient temperature.

The compounds of the formulae (X) and (XI) are prepared by standard methods known to the skilled man such as the methods of the Examples hereinafter, or by methods analogous or similar thereto.

Suitably, in the compounds of the formula (VIII), R¹⁴, R¹⁵ and R¹⁶ are independently selected from C₁₋₆ alkoxy such as methoxy, ethoxy, isopropoxy, n-propoxy or n-butoxy; aryloxy such as optionally substituted phenoxy; di-C₁₋₆alkylamino such as dimethylamino or diethylamino; diarylamino such as diphenylamino or any two of R¹⁴⁻R¹⁶ represent o-phenylenedioxy. Preferably each of R¹⁴⁻R¹⁶ have the same value and are C₁₋₆alkoxy for example methoxy, ethoxy, isopropoxy or n-butoxy or are phenoxy.

The compounds of the formula (VIII) are cyclized under conventional conditions known in the art to form compounds of the formula (V). Typical conditions are heating in a substantially inert organic solvent such as toluene, xylene or ethyl acetate at temperatures in the region 60-150°C. Typically the reaction is performed in an atmosphere of nitrogen and is carried out in the presence of a radical scavenger for example hydroquinone.

The compounds of the formula (VIII) may be formed and cyclized in situ. The compounds of the formula (VIII) may conveniently be prepared by reacting compounds of the formulae (XII) and (XIII):

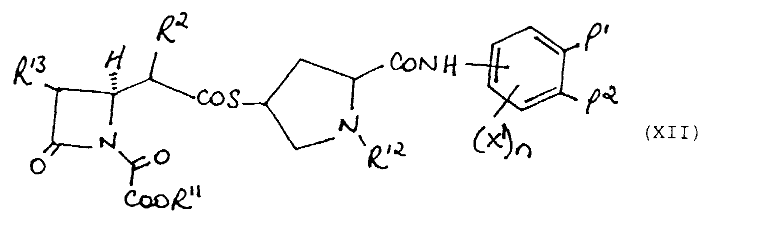

PR¹⁴R¹⁵R¹⁶ (XIII)

wherein R², X¹, n, R¹¹⁻R¹⁶, P¹ and P² are as hereinbefore defined. Suitably the compound of the formula (XIII) is a phosphite or is the functional equivalent of such a compound.

The reaction between the compounds of the formulae (XII) and (XIII) is conveniently performed in an organic solvent such as toluene, xylene, ethyl acetate, chloroform, dichloromethane, acetonitrile or dimethylformamide. Typically the reaction is carried out at an elevated temperature for example 60-150°C.

The compounds of the formula (XII) may be prepared by a number of methods known in the art. For example the compounds of the formula (XII) may be prepared by the acylation of a compound of the formula (XIV): 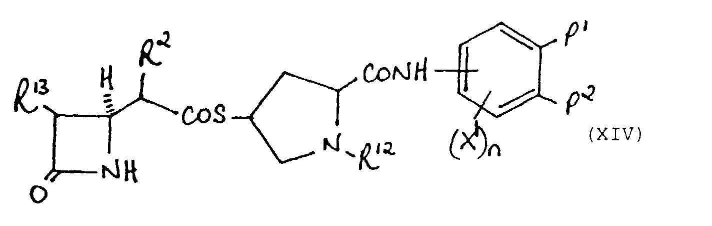 wherein R², R¹², R¹³, X¹, n, P¹ and P² are as hereinbefore defined with a compound of the formula (XV):

Cl-CO-COOR¹¹ (XV)

wherein R¹¹ is as hereinbefore defined.

The compounds of the formula (XIV) may be prepared by reacting compounds of the formulae (XVI) and (VII): 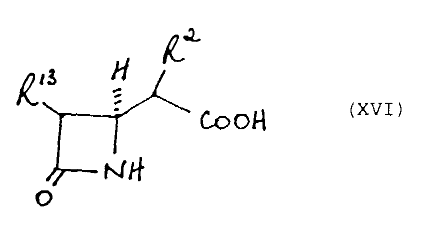 wherein R², X¹, n, R¹², R¹³, P¹ and P² are as hereinbefore defined. The compounds of the formula (XVI) are known in the art and may be reacted with compounds of the formula (VII) under conventional acylation conditions known in the art.

The following biological test methods, data and Examples serve to illustrate the present invention.

### Antibacterial Activity

The pharmaceutically acceptable carbapenem compounds of the present invention are useful antibacterial agents having a broad spectrum of activity in vitro against standard laboratory microorganisms, both Gram-negative and Gram-positive, which are used to screen for activity against pathogenic bacteria. The antibacterial spectrum and potency of a particular compound may be determined in a standard test system. In particular the carbapenems of the present invention show good stability to beta-lactamases and haveparticularlj high activity in vitro against strains of Pseudomonas aeruginosa and other Gram-negative aerobic bacteria.

The antibacterial properties of the compounds of the invention may also be demonstrated in vivo in conventional tests.

Carbapenem compounds generally have been found to be relatively non-toxic to warm-blooded animals, and this generalisation holds true for the compounds of the present invention. Compounds representative of the present invention were administered to mice at doses in excess of those required to afford protection against bacterial infections, and no overt toxic symptoms or side effects attributable to the administered compounds were noted.

The following results were obtained for representative compounds on a standard in vitro test system using Diagnostic Sensitivity Test agar medium. The antibacterial activity is described in terms of the minimum inhibitory concentration (MIC) determined by the agar-dilution technique with an inoculum size of 10⁴ CFU/spot.

| | MIC (µg/ml) |
|---|---|
| ORGANISM | EXAMPLE 2 |
| P.aeruginosa PU21 (101028) | 0.25 |
| | |
| Ent.cloacae P99⁻ (401054) | 0.015 |
| | |
| Serr.marcesens (421003) | 0.125 |
| | |
| Pr.morganii (433001) | 0.125 |
| | |
| E.coli DCO (341098) | 0.015 |
| | |
| St.aureus 147N (601052) | 0.25 |
| | |
| S.dublin (369001) | 0.008 |
| | |
| Strep.pyogenes (681018) | 0.008 |
| | |
| P. aeruginosa 18S (101024) | 0.06 |
| | |
| P. aeruginosa 18S IMIR(101148) | 0.06 |
| | |
| B. fragilis AMP R (542008) | 0.25 |

### Example 1

### (5R,6S,8R,2'S,4'S)-2-(2-(3,4-Dihydroxyphenylcarbamoyl)pyrrolidin-4-yl-thio)-6-(1-hydroxyethyl)carbapenem-3-carboxylic acid.

A solution of p-nitrobenzyl (5R,6S,8R,2'S,4'S)-2-(1-allyloxycarbonyl-2-(3,4-diallyloxyphenylcarbamoyl)pyrrolidin-4-yl-thio)-6-(1-hydroxyethyl)carbapenem-3-carboxylate (0.5mM) and dimedone (1.5mM) in THF (14 ml) was purged with argon. A solution of tetrakis(triphenylphosphine)palladium (0.05mM) in THF was added and the mixture was stirred under argon for 1 hour.

4-Morpholinopropane-3-sulphonic acid buffer (15 ml) was added, followed by 10% palladium on carbon (350 mg) and the mixture was hydrogenated at atmospheric pressure for 1 hour. The mixture was filtered, THF removed by evaporation, and the filtrate was washed with ethyl acetate prior to medium pressure chromatography on HP20SS resin (gradient elution with aqueous acetonitrile). The desired fractions were collected, evaporated to remove acetonitrile and freeze dried to give the title compound (12%): NMR 1.11(d, 3H); 1.83(partially obscured); 2.71(m, 1H); 2.98(q, 1H); 3.20(d, 2H); 3.23(q, 1H); 3.53(q, 1H); 3.78(m, 1H); 3.92(m, 1H); 4.04(t, 1H); 4.10(d of t, 1H); 6.63(d, 1H); 6.75(d, 1H); 7.18(s, 1H).

### Example 2

### (1R,5S,6S,8R,2'S,4'S)-2-(2-(5-Cyano-3,4-dihydroxyphenylcarbamoyl)pyrrolidin-4-ylthio)-6-(1-hydroxyethyl)-1-methyl-carbapenem-3-carboxylic acid.

To a solution of allyl (1R,5S,6S,8R,2'S,4'S)-2-(1-allyloxycarbonyl-2-(3,4-diallyloxy-5-cyanophenylcarbamoyl))pyrrolidin-4-yl-thio)-6-(1-hydroxyethyl)-1-methyl-carbapenem-3-carboxylate (0.25mM) and Meldrum's acid (2.0mM) in DMF (1.3 ml), under an argon atmosphere, was added a solution of tetrakis(triphenylphosphine)palladium (0.025mM) in THF (0.2 ml). The solution was stirred, under argon with protection from the light, for 25 minutes. THF (10 ml) was added slowly to precipitate the product. The resultant suspension was stirred for 10 minutes, the product was collected by filtration, washed with tetrahydrofuran and ether and dried to give the title product (66%): NMR 1.11(2d, 6H); 1.91(m, 1H); 2.88(m, 1H); 3.20(m, 2H); 3.35(m, 1H); 3.75(m, 1H); 3.96(m, 2H); 4.19(d of d, 1H); 4.32(t, 1H); 7.23(d, 1H); 7.32(d, 1H): m/s +ve FAB (M+H)⁺ = 489.

### Examples 3-12

By the general method of Example 2, the following reactions were performed:

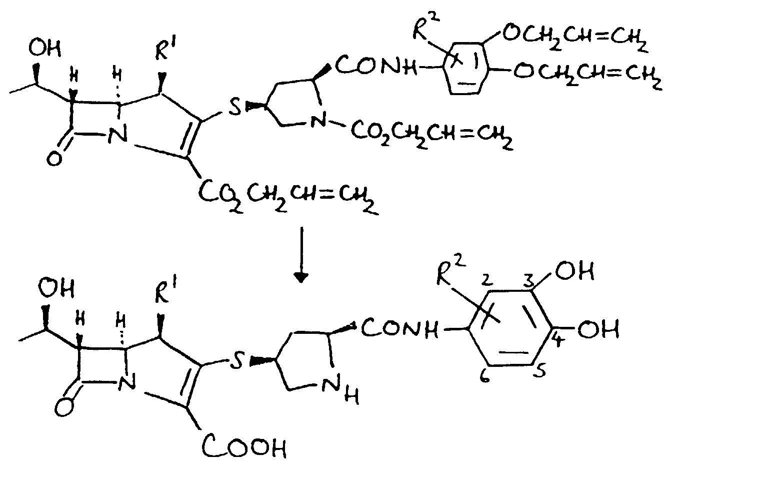

**Table 1**

| Example | R¹ | R² | Reaction Conditions | | |
|---|---|---|---|---|---|
| | | | Solvent | Ratio Carbapenem: Meldrums Acid | Time (mins) |
| 3 | H | 5-Br | THF(6 ml) | 0.45mM : 1.8mM | 60 |
| 4 | CH₃ | H | DMF(1.5 ml) | 0.25mM : 2.0mM | 120 |
| 5 | CH₃ | 5-OCH₃ | DMF(1.5 ml) | 0.25mM : 2.0mM | 25 |
| 6 | CH₃ | 2,5-di-Cl | THF(3 ml) | 0.2mM : 1.6mM | 10 |
| 7 | CH₃ | 5-OH | DMF(0.5 ml) | 0.28mM : 2.8mM | 45 |
| (from the corresponding 5-OCH₂CH=CH₂ compound) | | | | | |
| 8 | CH₃ | 5-Br | DMF(0.5 ml) | 0.27mM : 2.7mM | 60 |
| 9 | CH₃ | 5-CH=NOCH₃ | DMF(0.25 ml) | 0.12mM : 1.2mM | 50 |
| 10 | CH₃ | 6-COOCH₃ | DMF(0.28ml) | 0.28mM : 2.7mM | 105 |
| 11 | CH₃ | 6-COOH | DMF(0.3ml) | 0.21mM : 2.13mM | 75 |
| (from the corresponding 6-COOCH₂CH=CH₂ compound) | | | | | |
| 12 | CH₃ | 6-SO₂N(CH₂CH₃)₂ | THF/DMF (.1ml/.2ml) | 0.23Mm : 2.43Mm | 160 |
| FOOTNOTES: | | | | | |
| In Examples 3 and 6 the product precipitated spontaneously from the reaction mixture. The amount of palladium-phosphine catalyst was between 0.1-0.2 equivalents compared to the carbapenem. | | | | | |
| In Example 12 dilution of the reaction with THF(10 mL) failed to give a precipitate. Therefore the product was purified as follows: The solvent was evaporated. Ether was added to the residue to give a precipitate which was triturated with ether and THF. The impure solid was dissolved in 3.5 ml of water and 1.5 ml of acetonitrile. This solution was extracted with ethyl acetate. The aqueous layer was freeze dried to give the product as a white solid. | | | | | |

### NMR and MS Data for Examples 3-12 (Table 1)

- Example 3:: NMR: 1.11(d, 3H); 1.88(m, 1H); 2.79(m, 1H); 3.12(m, 1H); 3.21(m, 2H); 3.25(m, 1H); 3.65(m, 1H); 3.87(m, 1H); 3.93(m, 1H); 4.12(m, 1H); 4.20(m, 1H); 7.11(d, 1H); 7.21(d, 1H).
- Example 4:: NMR: 1.10(2d, 6H); 1.79(m, 1H); 2.78(m, 1H); 3.03(d of d, 1H); 3.20(d of d, 1H); 3.37(m, 1H); 3.61(m, 1H); 3.82(m, 1H); 3.95(m, 1H); 4.15(m, 2H); 6.65(d, 1H); 6.80(d of d, 1H); 7.15(d, 1H): M/S +ve FAB (M+H)⁺ = 464.
- Example 5:: NMR: 1.11(2d, 6H); 1.89(m, 1H); 2.85(m, 1H); 3.20(m, 2H); 3.35(m, 1H); 3.71(s, 3H); 3.74(m, 1H); 3.94(m, 2H); 4.18(d of d, 1H); 4.30(d of d, 1H); 6.72(d, 1H); 6.78(d, 1H): M/S +ve FAB (M+H)+ = 494.
- Example 6:: NMR: 1.12(2d, 6H); 1.9(partially obscured); 2.8(m, 1H); 2.98(m, 1H); 3.2(m, 1H); 3.38(m, 1H); 3.63(m, 1H); 3.8(m, 1H); 3.95(m, 1H); 4.18(d of d, 1H); 4.25(t, 1H); 7.25(s, 1H).
- Example 7:: NMR: 1.12(2d, 6H); 1.78(partially obscured); 2.75(m, 1H); 2.99(m, 1H); 3.20(dd, 1H); 3.37(m, 1H); 3.55(partially obscured); 3.8(m, 1H); 3.95(quintet, 1H); 4.08(t, 1H), 4.17(dd, 1H); 6.60(s, 2H).
- Example 8:: NMR: 1.12(2d, 6H); 1.70(m, 1H); 2.65(m, 1H); 2.85(m, 1H); 3.20(m, 1H); 3.45(m, 1H); 3.60(m, 1H); 3.70(m, 1H); 3.95(m, 2H); 4.15(m, 1H); 7.15(s, 1H); 7.24(s, 1H).
- Example 9:: NMR: 1.15(2d, 6H); 1.79(m, 1H); 2.75(m, 1H); 2.99(m, 1H); 3.20(dd, 1H), 3.39(m, 1H); 3.59(m, 1H); 3.80(m, 1H); 3.88(s, 3H); 3.95(quintet, 1H); 4.09(t, 1H); 4.19(dd, 1H); 7.22(s, 1H); 7.29(s, 1H); 8.32(s, 1H): M/S +ve FAB (M+H)⁺ = 521.
- Example 10:: NMR: 1.12(d, 6H); 1.64(m, 1H); 2.62(m, 1H); 3.19(dd, 1H); 3.30-3.48(partially obscured); 3.5(m, 1H); 3.79(s, 3H); 3.89(m, 1H); 3.95(m, 1H); 4.14(m, 1H); 7.35(s, 1H); 8.2(s, 1H): M/S +ve FAB (M+H)⁺ = 522.
- Example 11:: NMR: 1.12(d,6H); 1.68(m,1H); 2.53-2.66(m,2H); 3.18(dd,1H); 3.4(m,1H); 3.49(m,1H); 3.55(m,1H); 3.93(m,2H); 4.15(dd,1H); 7.35(s,1H); 8.12(m,1H): M/S +ve FAB (M+H)⁺=508.
- Example 12:: NMR: 1.03(t,6H); 1.18(2s,6H); 1.80(m,1H); 2.70(m,1H); 3.20(m,6H); 3.45(m,1H); 3.62(m,2H), 4.05(m,2H); 4.20(m,1H); 7.20(s,1H); 7.85(br,1H): M/S +ve FAB (M+H)⁺=599.

The starting materials for Examples 1 to 12 were prepared in the following general manner:

To a stirred solution of the appropriate 6-(1-hydroxyethyl)-2-oxocarbapenam-3-carboxylic acid ester or 6-(1-hydroxyethyl)-1-methyl-2-oxocarbapenam-3-carboxylic acid ester (1 equivalent) and diisopropylethylamine (1.1 equivalents) in acetonitrile at 0°C, under an argon atmosphere, was added dropwise diphenyl chlorophosphate (1.1 equivalents). The solution was stirred at ambient temperature for 30 minutes to form the corresponding 3-diphenylphosphoryloxycarbapenem.

The solution was cooled to 0° and di-isopropylethylamine (1.5 equivalents) and the appropriate side-chain pyrrolidin-4-yl mercaptan (1.3 equivalents) in acetonitrile were added. The resultant solution was stirred at 0°C for 90 minutes, solvent was removed by evaporation and the desired product isolated by medium pressure chromatography on silica (eluting with propan-2-ol/dichloromethane) to give an amorphous foam.

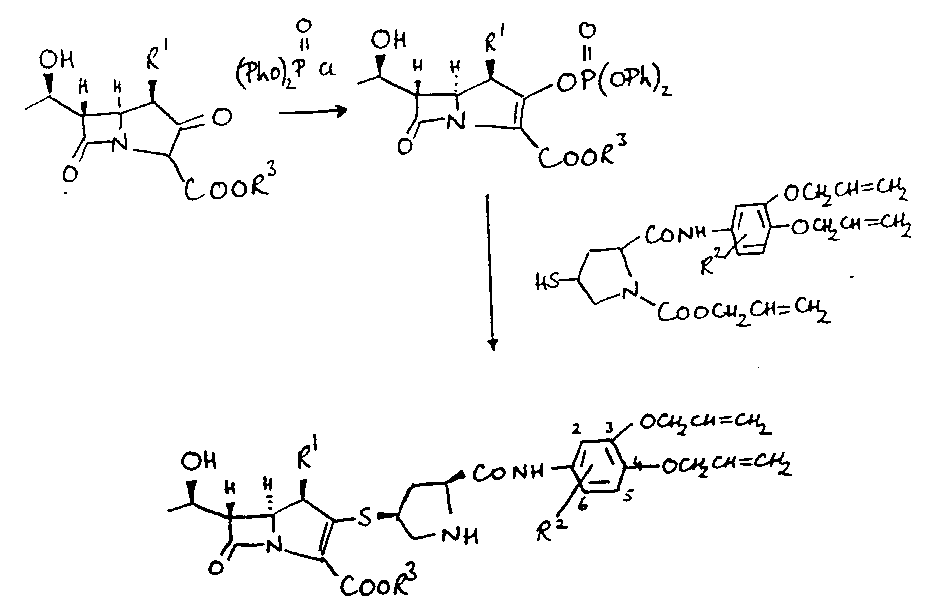

**Table 2**

| Preparation of starting material for Example | R¹ | R² | R³ | Footnotes |
|---|---|---|---|---|
| 1 | H | H | CH₂-Ph-NO₂(p) | |
| 2 | CH₃ | 5-CN | CH₂CH=CH₂ | 1 |
| 3 | H | 5-Br | CH₂CH=CH₂ | |
| 4 | CH₃ | H | CH₂CH=CH₂ | |
| 5 | CH₃ | 5-OCH₃ | CH₂CH=CH₂ | 2 |
| 6 | CH₃ | 2,5-di-Cl | CH₂CH=CH₂ | 2 |
| 7 | CH₃ | 5-OCH₂CH=CH₂ | CH₂CH=CH₂ | 2,3 |
| 8 | CH₃ | 5-Br | CH₂CH=CH₂ | 2,3 |
| 9 | CH₃ | 5-CH=NOCH₃ | CH₂CH=CH₂ | 2,4 |
| 10 | CH₃ | 6-COOCH₃ | CH₂CH=CH₂ | 2,5 |
| 11 | CH₃ | 6-COOCH₂CH=CH₂ | CH₂CH=CH₂ | 1,3 |
| 12 | CH₃ | 6-SO₂N(CH₂CH₃)₂ | CH₂CH=CH₂ | 3,6 |
| FOOTNOTES TO TABLE 2 | | | | |
| 1. Chromatography eluent: ethyl acetate. | | | | |
| 2. Chromatography: gradient elution with acetonitrile/ethyl acetate. | | | | |
| 3. Reaction for 16 hours at 0°C. | | | | |
| 4. Reaction for 6 hours at 0°C with warming to ambient. | | | | |
| 5. Reaction for 72 hours at 0°C. | | | | |
| 6. Chromatography: gradient elution ethyl acetate/hexane. | | | | |

### NMR and MS Data for Products of Table 2.

1. NMR(DMSO-d₆): 1.13(d, 3H); 1.88(m, 1H); 2.77(m, 1H); 3.3(partially obscured); 4.10(m, 6H); 4.48(m, 6H); 5.30(m, 8H); 6.02(m, 3H); 6.88(d, 1H); 7.07(d of d, 1H); 7.30(d, 1H); 7.69(d, 2H); 8.22(d, 2H): M/S +ve FAB (M+H)⁺ = 749.
2. NMR: 1.12(2d, 6H); 1.90(partially obscured); 2.75(m, 1H); 3.22(d of d, 1H); 3.31(d of d, 1H); 3.50(m, 1H); 3.89(m, 1H); 3.97(m, 1H); 4.07(m, 1H); 4.22(m, 1H); 4.50(m, 9H); 5.27(m, 8H); 5.90(m, 4H); 7.48(s, 1H); 7.60(s, 1H): M/S +ve FAB (M+H)⁺ = 693.
3. NMR: 1.04(d, 3H); 1.90(partially obscured); 2.92(m, 1H); 3.23(m, 3H); 3.97(m, 5H); 4.43(m, 9H); 5.20(m, 8H); 5.83(m, 4H); 7.23(s, 1H); 7.42(s, 1H): M/S +ve FAB (M+H)⁺ = 734.
4. NMR: 1.15(2d, 6H); 1.83(partially obscured); 2.73(m, 1H); 3.24(m, 1H); 3.51(m, 1H); 3.91(m, 2H), 4.10(m, 1H); 4.22(d, 1H); 4.50(m, 10H); 5.27(m, 8H); 5.90(m, 4H); 6.89(d, 1H); 7.08(d, 1H); 7.30(d of d, 1H): M/S +ve FAB (M+H)⁺ = 668.
5. NMR: 1.12(2d, 6H); 1.85(partially obscured); 2.74(m, 1H); 3.23(m, 2H); 3.50(m, 1H); 3.70(s, 3H); 3.86(m, 1H); 3.97(m, 1H); 4.08(d of d, 1H); 4.21(d, 1H); 4.43(m, 9H); 5.20(m, 8H); 5.90(m, 4H); 6.97(s, 2H): M/S +ve FAB (M+H)⁺ = 698.
6. NMR: 1.15(2d, 6H); 1.99(m, 1H); 2.79(m, 1H); 3.23(m, 1H); 3.31(m, 1H); 3.50(m, 1H); 3.91(m, 1H); 3.98(m, 1H); 4.08(m, 1H); 4.25(d of d, H); 4.54(m, 8H); 4.68(m, 1H); 5.1-5.4(m, 8H); 5.76-6.1(m, 4H); 7.69(br s, 1H): M/S +ve FAB (M+H)⁺ = 736.
7. NMR: 1.15(2d, 6H);, 1.85(partially obscured); 2.74(m, 1H); 3.22(d of d, 1H); 3.28(m, 1H); 3.50(m, 1H); 3.88(m, 1H); 3.99(m, 1H); 4.09(m, 1H); 4.20(d of d, 1H); 4.33-4.58(m, 10H); 4.65(m, 1H); 5.0-5.4(m, 10H); 5.7-6.09(m, 5H); 6.99(d, 2H): M/S +ve FAB (M+H)⁺ = 724.
8. NMR: 1.14(2d, 6H); 1.89(partially obscured); 2.75(m, 1H); 3.23(dd, 1H); 3.3(m, 1H); 3.50(m, 1H); 3.89(m, 1H); 3.98(quintet, 1H); 4.08(m, 1H); 4.22(d, 1H); 4.32-4.59(m, 8H); 4.68(m, 1H); 5.14-5.42(m, 8H); 5.72-6.1(m, 4H); 7.32(s, 1H); 7.49(s, 1H): M/S +ve FAB (M+H)⁺ = 746/748.
9. NMR: (DMSO-d₆): 1.15(2d, 6H); 1.82(m, 1H); 2.75(m, 1H); 3.25(partially obscured); 3.55(m, 1H); 3.90(s, 3H); 3.95(m, 1H); 4.10(m, 1H); 4.22 (m, 1H); 4.35-4.61(m, 8H); 4.69(m, 1H); 5.05(m, 1H); 5.18-5.45(m, 8H); 5.75-6.1(m, 4H); 7.22(m, 1H); 7.40(m, 1H); 8.25 (s, 1H); 10.05(br s, 1H).
10. NMR: 1.12(2d, 6H); 2.04(m, 1H); 2.82(m, 1H); 3.22(d of d, 1H); 3.45(m, 2H); 3.72(s, 3H); 3.98(m, 2H); 4.09(d of d, 1H); 4.25(dd, 1H); 4.39-4.65(m, 9H); 5.09-5.49(m, 8H); 5.68-6.1(m, 4H); 7.48(s, 1H); 8.35(d, 1H): M/S +ve FAB (M+H)⁺ = 726.
11. NMR: 1.12(d,6H); 2.02(m,1H); 2.81(m,1H); 3.22(dd,1H); 3.45(m,2H); 3.99(m,2H); 4.09(dd,1H); 4.24(dd,1H); 4.39-4.68(m,10H); 4.75(dd,1H); 5.1-5.49(m,10H); 5.69-6.1(m,5H); 7.5(s,1H); 8.35(br d,1H): M/S +ve FAB (M+H)⁺ = 752.
12. NMR: (DMSO-d₆): 0.98(m,6H); 1.15(m,6H); 2.00(m,1H; 2.80(m,1H); 3.15(m,4H); 3.25(m,1H); 3.50(m,1H); 3.95(m,2H); 4.20(m,2H); 4.40-4.70(m,9H); 5.05-5.50(m,9H); 5.70-6.10(m,4H); 7.19(s,1H); 8.15(br.d, 1H); 10.1(br d, 1H): M/S +ve FAB (M+H)⁺=803.

Esters of 6-(1-hydroxyethyl)-2-oxocarbapenam carboxylic acid and 6-(1-hydroxyethyl)-1-methyl-2-oxocarbapenam carboxylic acid are well known in the literature, see for example EP-A-126780 and EP-A-208889.

We prefer to prepare allyl 6-(1-hydroxyethyl)-1-methyl-2-oxocarbapenam carboxylate in situ from allyl 2-diazo-3-oxo-4-methyl-4-(3-(1-hydroxyethyl)-2-oxoazetidin-4yl)butanoate and rhodium octanoate (see for example EP-A-341557 and Tet. Lett. 1988, 29, 61).

### PREPARATION OF SIDE CHAIN PYRROLIDIN-4-YL MERCAPTANS

The side-chain pyrrolidin-4-yl mercaptans were prepared in the following general manner:

4-Acetylthio-1-allyloxycarbonyl-2-carboxypyrrolidine (1 equivalent) was treated with oxalyl chloride (2 equivalents) in dichloromethane in the presence of a small amount of DMF. The reaction mixture was stirred at ambient temperature for 30 minutes, a further drop of DMF was added, and stirring was continued for a further 20 minutes. The mixture was evaporated (azeotroping with toluene) and filtered to remove Vilsmeier reagent. The resultant acid chloride was dissolved in THF and added dropwise to the appropriate aniline (generated in situ from the corresponding hydrochloride) in THF in the presence of diisopropylethylamine. The mixture was stirred for 60-150 minutes at 0°C, under argon, diluted with ethyl acetate, washed (H₂O, 2N HCl, H₂O, saturated NaHCO₃), evaporated and subjected to medium pressure chromatography with ethyl acetate/hexane to give the corresponding 4-acetylthio-1-allyloxycarbonyl-2-(3,4-diallyloxyphenylcarbamoyl)pyrrolidine (compound C).

The 4-acetylthio compound was dissolved in methanol and the solution flushed with argon. 1N Sodium hydroxide (1.1-1.5 equivalents) was added and the mixture was stirred at ambient temperature for 60 minutes. 2N Hydrochloric acid (1.1-1.5 equivalents) was added, methanol removed by evaporation and the residue partitioned between ethyl acetate and water. The organic layer was washed with brine, dried (MgSO₄) and evaporated to give the corresponding 1-allyloxycarbonyl-2-(3,4-diallyloxyphenylcarbamoyl)pyrrolidin-4-yl thiol (compound D) as a gum.

Thus the following compounds were prepared according to the following Schemes:

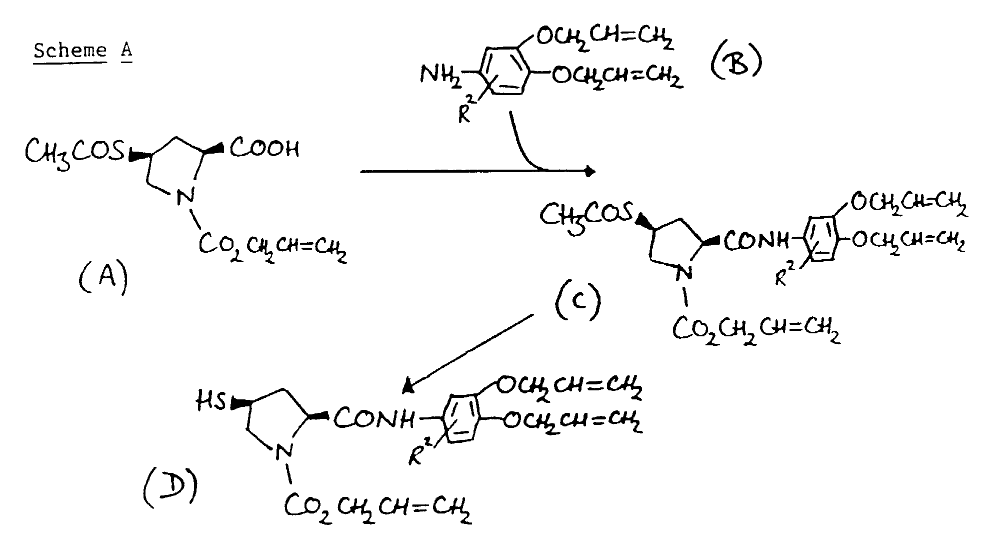

### NMR and MS Data for compounds (D).

R=H (CDCl₃) 1.88(d, 1H); 2.47(br s, 1H); 2.64(br s, 1H); 3.36(m, 2H); 4.07(m, 1H); 4.47(t, 1H); 4.59(m, 6H); 5.32(m, 6H); 5.99(m, 3H); 6.81(d, 1H); 6.88(d of d, 1H); 7.32(d, 1H); 8.71 (br s, 1H): M/S +ve FAB (M+H)⁺ = 419.
R=5-Br (DMSO-d₆) 1.79(d, 1H); 2.68(m, 1H); 3.05(m, 1H); 3.2(partially obscured); 3.95(m, 1H); 4.28(m, 1H); 4.42-4.6(m, 6H); 5.0-5.48(m, 6H); 5.69-6.17(m, 3H); 7.32(d, 1H); 7.50(d, 1H); 10.05(s, 1H).
R=2,5-di-Cl (DMSO-d₆) 1.88(m, 1H); 2.72(m, 1H); 3.01(dd, 1H); 3.38(m, 1H); 3.95(m, 1H); 4.42-4.6(m, 7H); 5.08-5.45(m, 6H); 5.75-6.15(m, 3H); 7.6(br d, 1H); 9.62(br s, 1H): M/S +ve FAB (M+H)⁺ = 487.
R=5-OCH₂CH=CH₂ (DHSO-d₆) 1.80(m, 1H); 2.68(m, 1H); 3.08(m, 1H); 3.38(partially obscured); 3.95(m, 1H); 4.24-4.55(m, 9H); 5.02-5.48(m, 8H); 5.9-6.15(m, 4H); 6.99(d, 2H); 9.91(br s, 1H).
R=5-CH=NOCH₃ 1.8(partially obscured); 2.70(m, 1H); 3.22(m, 1H); 3.38(m, 1H); 3.90(s, 3H); 3.98(partially obscured); 4.32(m, 1H); 4.45-4.6(m, 6H); 5.15-5.5(m, 6H); 5.85-6.18(m, 3H); 7.55(m, 2H); 8.30(s, 1H).
R=6-CO₂CH₃ NMR (at 80°C) 2.24(m, 1H); 2.72(m, 1H); 3.6(m, 2H); 3.84(d, 3H); 4.02(m, 1H); 4.36-4.68(m, 7H); 5.06-5.5(m, 6H); 5.78-6.18(m, 3H); 7.53(d, 1H); 8.32(d, 1H): M/S + ve FAB (M+H)⁺ = 477.
R=6 COOCH₂CH=CH₂ NMR 1.91(m,1H); 2.72(m,2H); 3.43(m, partially obscured); 3.90(m,1H); 4.04(m,1H); 4.33(m,1H); 4.42-4.82(m,8H); 5.0-5.52(m,9H); 5.72-6.18(m,4H); 7.49(d,1H); 8.3(m,1H): M/S +ve FAB (M+H)⁺ = 503.
[The hydrolysis of this compound was performed using allyl alcohol as solvent].
R=6-SO₂N(CH₂CR₃)₂ NMR (DMSO-d₆): 0.99(m,6H); 2.19(m,1H); 2.71(m,1H); 3.00-3.56(m,6H); 3.72(m,1H); 3.98(m,1H); 4.30-4.65(m,7H); 5.10-5.51(m,6H); 5.64-6.15(m,3H); 7.18(d,1H); 8.20(br,1H); 10.1(br,1H): M/S +ve FAB (M+H)⁺=554.
Compounds D wherein R=5-CN and 5-OCH₃ were prepared and utilised in situ without characterisation.

### NMR and MS Data for Compounds (C)

R=H (CDCl₃) 2.32(s,3H); 2.58(br s,2H); 3.36(q,1H); 4.06(m,2H); 4.58(m,7H); 5.32(m,6H); 6.00(m,3H); 6.81(d,1H); 6.90(d of d,1H); 7.32(d,1H); 8.85(br s, 1H): M/S +ve FAB (M+H)⁺=461.
R=5-Br (DMSO-d₆) 1.88(m,1H); 2.34(s,3H); 2.76(m,1H); 3.25(partially obscured); 3.98(m,2H); 4.35(m,1H); 4.45-4.6(m,6H); 5.02-5.49(m,6H); 5.72-6.18(m,3H); 7.3(d,1H); 7.5(d,1H); 10.05(br s,1H).
R=2,5-di-Cl (DMSO-d₆) 1.94(m,1H); 2.33(s,3H); 2.76(m,1H); 3.99(m,2H); 4.55(m,7H); 5.05-5.45(m,7H); 5.78-6.18(m,3H); 7.59(br d,1H); 9.68(br s,1H): M/S +ve FAB (M+H)⁺ = 529.
R=5-OCH₂CH=CH₂ (DMSO-d₆) 1.85(m,1H); 2.32(s,3H); 2.75(m,1H); 4.0(m,2H); 4.3-4.58(m,9H); 5.02-5.48(m,9H); 5.7-6.15(m,4H); 6.98(s,2H); 9.91(s,1H).
R=5-CH=NOCH₃ (DMSO-d₆) 1.88(m,1H); 2.32(s,3H); 2.75(m,1H); 3.32(partially obscured); 3.9(s,3H); 4.0(m,2H); 4.3-4.59(m,7H); 5.02-5.49(m,6H); 5.75-6.17(m,3H); 7.45(d,1H); 7.54(br d,1H); 8.28(s,1H); 10.05(s,1H).
R=6-COOCH₂CH=CH₂ (DMSO-d₆) 1.95(m,1H); 2.32(s,3H); 2.78(m,1H); 3.25 and 3.96 (partially obscured); 4.3(m,1H); 4.4-4.85(m,8H); 5.1-5.5(m,8H); 5.8-6.15(m,4H); 7.52(s,1H); 8.3(br s,1H).
R=5-CN (CDCl₃) 2.34(s,3H); 2.50(m,2H); 3.39(q,1H); 4.08(m,2H); 4.58(m,7H); 5.32(m,6H); 6.00(m,3H); 7.14(d,1H); 7.53(d,1H); 9.38(br s, 1H): M/S +ve FAB (M+H)⁺= 486.
R=5-OCH₃ (CDCl₃) 2.33(s,3H); 2.53(m,2H); 3.37(q,1H); 3.82(s,3H); 4.07(m,2H); 4.50(m,5H); 4.66(d,2H); 5.27(m,6H); 6.00(m,3H); 6.81(s,2H); 9.04(br s,1H): M/S +ve FAB (M+H)⁺=491.
R=6-SO₂N(CH₂CH₃)₂ (DHSO-d₆): 1.00(t,6H); 2.03(m,1H); 2.32(s,3H); 2.79(m,1H); 3.14-3.32(m, partially obscured); 4.05(m,2H); 4.40-4.65(m,7H); 5.16-5.50(m,6H); 5.80-6.15(m,3H); 7.19(s,1H); 8.15(br s,1H); 10.10(br s,1H): M/S +ve FAB (M+H)⁺=596.

### Compound (A)

4-Acetylthio-1-allyloxycarbonylpyrrolidine-2-carboxylic acid was prepared as follows:
a) To a solution of 4-hydroxyproline (0.25M) in 4N sodium hydroxide (78ml), at 0°-5°C, was added allyl chloroformate (0.31M) in dioxan (156ml) and 4N sodium hydroxide (78ml). The dioxan solution and sodium hydroxide were added simultaneously, in batches, allowing the temperature to return to below 5°C after each addition. The mixture was stirred for a further hour, washed with ethyl acetate, acidified (conc. HCl) and extracted into ethyl acetate. The organic phase was washed with brine, dried and evaporated to give 1-allyloxycarbonyl-4-hydroxypyrrolidine-2-carboxylic acid as a viscous syrup. This (0.1M) was treated with 4-methoxybenzyl chloride (0.19M) and triethylamine (0.2M) in DMF (200ml), under argon, at 70°C for 10 hours. Ethyl acetate was added and the mixture was washed with water and brine and evaporated to give a gum. This was subjected to medium pressure chromatography (gradient elution with ether to ether/ 1% methanol) to give 4-methoxybenzyl 1-allyloxycarbonyl-4-hydroxypyrrolidine-2-carboxylate.
b) To a solution of the product from (a) (0.066M) and triphenylphosphine (0.099M) in THF (200ml) at 0°C, under argon, was slowly added diethylazodicarboxylate (0.099M) in THF (60ml). The solution was stirred for 30 minutes at 0°C. Thiolacetic acid (0.099M) in THF (60ml) was slowly added and the resultant mixture was stirred for 1 hour at 0°C, for 18 hours at ambient, evaporated and subjected to medium pressure chromatography (ethyl acetate/hexane gradient elution) to give 4-methoxybenzyl 4-acetylthio-1-allyloxycarbonylpyrrolidine-2-carboxylate.
c) To the product from (b) above (0.056M) was added anisole (0.112M) in trifluoroacetic acid (60ml) at ambient temperature. The mixture was stirred for 15 minutes, evaporated, and the residue was dissolved in ether (150ml) and treated with cyclohexylamine (0.056M) in ether (50ml). The cyclohexylamine salt of 4-acetylthio-1-allyloxycarbonylpyrrolidine-2-carboxylic acid crystallised out and was collected: NMR (CDCl₃) 2.0(m,1H); 2.30(s,3H); 2.56(m,1H); 3.32(dd,1H); 3.91(m,1H); 4.05(dd,1H); 4.16(t,1H); 4.53(m,2H); 5.20(m,2H); 5.90(m,1H); 6.68(br s, 3H) and cyclohexylamine peaks at 1.0-2.0 and 2.95: M/S C.I. (M+H)⁺ = 274.

### Compounds (B) in Examples 1-11

Compounds (B) in examples 1-11 were prepared according to the following Scheme:

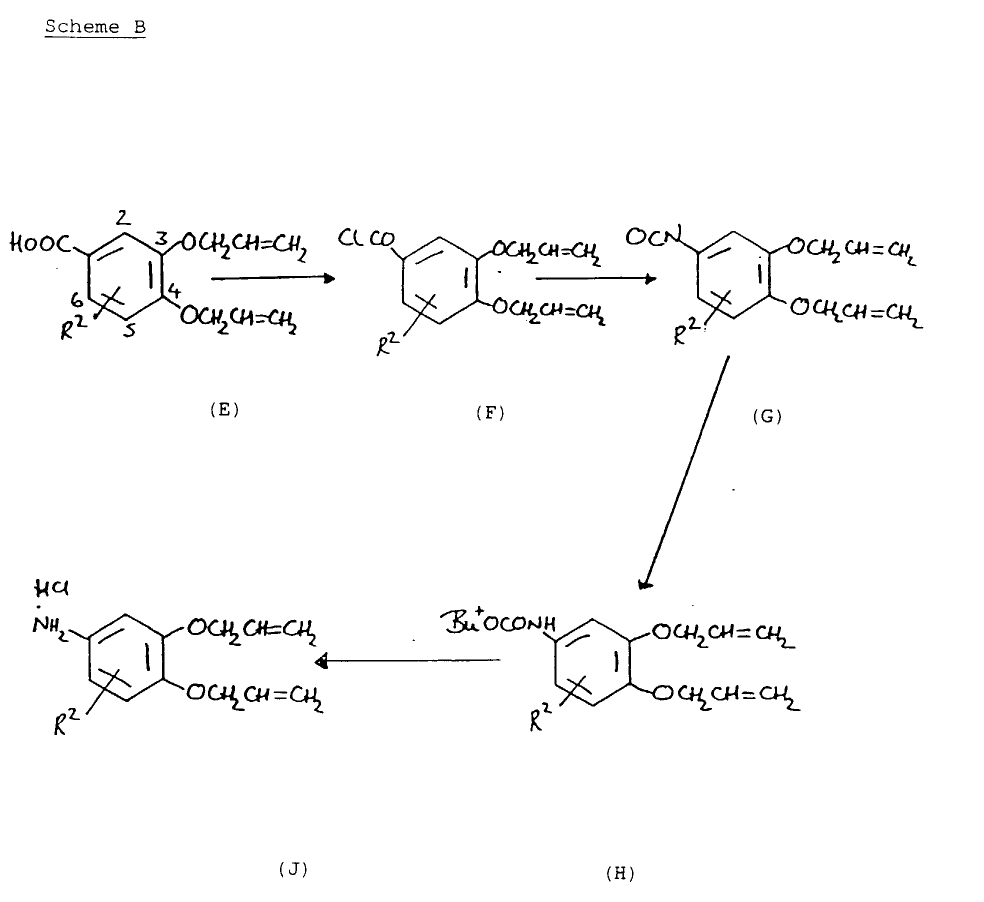

### General method of preparation of Compounds (J):

Acid (E) (1.73mmol) in dichloromethane (5ml) was treated with oxalyl chloride (2.59mmol) and DMF (one drop). The mixture was stirred, under argon, for 30 minutes at room temperature. Further DMF (one drop) was added, the mixture stirred for a further 30-60 minutes, evaporated (azeotroping with toluene), dissolved in toluene, filtered and evaporated to afford the acid chloride (F).

The acid chloride (F) (1.73mmol) and trimethylsilyl azide (2.59mmol) were heated at reflux in toluene (5ml) for 3-18 hours. The mixture was evaporated to give the isocyanate (G) as an oil which was used without purification. The isocyanate (1.73mmol) was heated at reflux in t-butanol (5ml) for 1/2 - 3 hours, evaporated and subjected to medium pressure chromatography (ethyl acetate/hexane) to give compound (H). This was dissolved in a minimum amount of ethyl acetate and treated with ethyl acetate saturated with HCl to give the aniline hydrochloride (J) as a precipitate.

All reactions were followed by thin layer chromatography.

### NMR data for Compounds (J)

R²=2, 5-di-Cl (DMSO-d₆) 4.39(m,2H); 4.52(m,2H); 5.19-5.44(m,4H); 5.95-6.15(m,2H); 6.68(s,1H): M/S (M+H)⁺=274.
R²=5-OCH₂CH=CH₂ (DMSO-d₆) 4.44(m,2H); 4.55(s,4H); 5.12-5.49(m,6H); 5.91-6.15(m,3H); 6.65(s,2H).
R²=5-CH=NOCH₃(DMSO-d₆) 3.92(s,3H); 4.5(dt,2H); 5.2-5.5(m,4H); 5.92-6.19(m,2H); 7.02(d,1H); 7.19(d,1H); 8.29(s,1H).
R²=H (DMSO-d₆) 4.56(m,4H); 5.33(m,4H); 6.06(m,2H); 6.90(d of d,1H); 7.03(d,1H); 7.08(d,1H).
R²=5-Br (DMSO-d₆) 4,48(m,2H); 4.61(m,2H); 5.32(m,4H); 6.05(m,2H); 6.92(d,1H); 7.03(d,1H).
R²=5-CN (DHSO-d₆) 4.62(m,4H); 5.32(m,4H); 6.03(m,2H); 6.97(d,1H); 7.16(d,1H): C.I. (H+H)⁺=231.
R²=5-OCH₃ (DHSO-d₆) 3.78(s,3H); 4.42(m,2H); 4.57(m,2H); 5.80(m,4H); 6.02(m,2H); 6.69(s,2H): C.I. (M+H)⁺=236.
R²=6-COOCH₂CH=CH₂ (DHSO-d₆) 4.45-4.78(3d,6H); 5.2-5.5(m,6H); 5.92-6.16(m,3H); 6.72(s,1H); 7.32(s,1H): M/S (M+H)⁺=290.
[This compound was prepared by acetylating 2-amino-4,5-dimethoxybenzoic acid to form 2-acetamido-4,5-dimethoxybenzoic acid; removing the methoxy groups with BBr₃; alkylating with allyl bromide and hydrolysing the acetamido group with conc. HCl in methanol]

### Preparation of Acids (Compounds (E))

3,4-Diallyloxy-5-bromobenzoic acid was prepared as follows:

Methyl 3,4-dihydroxy-5-bromobenzoate (49 g, see Chem. Abs., 89:2327g) and K₂CO₃ (92 g) were placed in a flask in acetone (500 ml). Allyl bromide (49 ml) was slowly added to the mixture, which was stirred at room temperature for 5 days. After filtration, solvent was evaporated, the residue taken up in ethyl acetate, washed with water, and dried (MgSO₄). Evaporation of the solvent gave methyl 3,4-diallyloxy-5-bromobenzoate (65 g): NMR (DHSO-d₆) 3.86 (s, 3H); 4.67 (m, 4H); 5.20-5.53 (m, 4H); 5.95-6.19 (m, 2H); 7.54 (d, 1H); 7.73 (d, 1H).

Crude ester (65 g) was dissolved in methanol (400 ml), and treated with aqueous sodium hydroxide (40 ml, 10.5 N). After reflux overnight, methanol was evaporated, the aqueous residue diluted with water, and acidified with conc. HCl. The white precipitate was filtered off, and dissolved in ethyl acetate. A water layer was separated, and the organic layer dried over MgSO₄. Filtration and evaporation gave as a white solid 3,4-diallyloxy-5-bromobenzoic acid (59 g): NMR (CDCl₃) 4.67 (m, 4H); 5.42-5.51 (m, 4H); 6.00-6.21 (m, 2H); 7.56 (d, 1H); 7.94 (d, 1B), 11.33 (s,1 1H).

2,5-Dichloro-3,4-diallyloxybenzoic acid was prepared as follows:

2,5-Dichloro-3,4-dihydroxybenzoic acid (10.7 g, see J. Antibiotics, 1987, 40, 22) and K₂CO₃ (33.1 g) were suspended in DMF (150 ml), and treated with allyl bromide (14.7 ml). After stirring overnight, the mixture was filtered, diluted with ethyl acetate, washed with sodium bicarbonate, dried (MgSO₄), filtered and evaporated to give allyl 2,5-dichloro-3,4-diallyloxybenzoate (14.7 g): NMR (CDCl₃) 4.55-4.65 (m, 4H); 4.82 (m, 2H); 5.24-5.50 (m, 6H); 5.94-6.22 (m, 3H); 7.70 (s, 1H).

Crude ester was hydrolysed as for the methyl ester above, to give 2,5-dichloro-3,4-diallyloxybenzoic acid (12 g): NMR (DMSO-d₆) 4.55-4.68 (m, 4H); 5.23-5.48 (m, 4H); 6.00-6.20 (m, 2H); 7.69 (s, 1H).

3,4,5-Triallyloxybenzoic acid was prepared as follows.

Ethyl gallate (7.92 g, commercially available, Fluka), and K₂CO₃ (27.6 g) in DMF (40 ml) were treated with allyl bromide (13.5 ml) as above, to give ethyl 3,4,5-triallyloxybenzoate (14 g): NMR (CDCl₃) 1.49 (t, 3H); 4.35 (q, 2H); 4.62 (m, 6H); 5.15-5.48 (m, 6H); 6.00-6.28 (m, 3H); 7.29 (s, 1H). This crude ester was hydrolysed essentially as above to give 3,4,5-triallyloxybenzoic acid(10.4 g): NMR (CDCl₃) 4.64 (m, 6H); 5.15-5.51 (m, 6H); 6.00-6.20 (m, 3H); 7.35 (s, 1H).

3,4-Diallyloxy-5-cyanobenzoic acid was prepared as follows:

3,4-Dihydroxy-5-cyanobenzoic acid, (see GB 2 200 109) was converted, essentially as above, to allyl 3,4-diallyloxy-5-cyanobenzoate: NMR (CDCl₃) 4.63 (m, 2H); 4.77-4.87 (m, 4H); 5.23-5.51 (m, 6H); 5.92-6.18 (m, 3H); 7.76 (d, 1H); 7.86 (d, 1H). This ester was hydrolysed as above to give 3,4-diallyloxy-5-cyanobenzoic acid: (DHSO-d₆) 4.72-4.84 (m, 4H); 5.23-5.51 (m, 4H); 5.93-6.18 (m, 2H); 7.79 (s, 2H).

3,4-Diallyloxy-5-methoxybenzoic acid was prepared as follows:

3,4-Dihydroxy-5-methoxybenzoic acid (50mM), K₂CO₃ (300mM) and allyl bromide (200mM) in DMF (100ml) was stirred vigorously at 80°C for 2 1/2 hours. The mixture was filtered and the filtrate partitioned between ethyl acetate and water. The organic phase was washed with brine and evaporated to give allyl 3,4-diallyloxy-5-methoxybenzoate as a crude oil: NMR (CDCl₃) 3.89(s,3H); 4.61(m,4H); 4.82(m,2H); 5.31(m,6H); 6.06(m,3H); 7.32(s,2H).

The ester was hydrolysed with KOH in water at 100°C for 45 minutes to afford 3,4-diallyloxy-5-methoxybenzoic acid, after chromatography, as a waxy solid; NMR (DHSO-d₆) 3.68(s,3H); 4.43(d of d,4H); 5.21(m,4H); 5.95(m,2H); 7.22(s,2H).

3,4-Diallyloxy-5-methoxyiminomethylbenzoic acid was prepared as follows:

2,3-Dihydroxy-5-carboxybenzaldehyde (GB-A-2200109) was alkylated with allyl bromide, as above, to give the corresponding tri-allyl derivative. This was treated with O-methylhydroxylamine hydrochloride and pyridine in ethanol at reflux to give allyl 3,4-diallyloxy-5-methoxyiminomethylbenzoate which was hydrolysed with KOH in aqueous ethanol at reflux to give
3,4-diallyloxy-5-methoxyiminomethylbenzoic acid; NMR (DMSO-d₆) 3.92(s,3H); 4.61(m,2H); 4.7(m,2H); 5.2-5.5(m,4H); 5.94-6.18(m,2H); 7.56(d,1H); 7.92(d,1H); 8.32(s,1H); 12.9(s,1H).

### Compound (B) in Example 12.

Condensed diethylamine (4.8 ml) was slowly added to a solution of 4,5-dimethoxy-2-nitrobenzenesulphonyl chloride*. The reaction mixture was stirred for 1 hour at 0°C and then heated on a steam bath for 10 minutes. The reaction was chilled, filtered and the solvent evaporated. The di-[N-ethyl]-4,5-dimethoxy-2-nitrobenzenesulphonamide thus prepared was purified by sinter flash chromatography eluting with dichloromethane (50%).
NMR (DHSO-d₆): 1.09(t,6H); 3.32(m,4H); 3.95(2xs, 6H); 7.25(s, 1H); 7.59(s, 1H): M/S CI (M+H)⁺=319.
* [4,5-dimethoxy-2-nitrobenzenesulphonyl chloride was prepared according to J. Med. Chem., 11, 136, (1968).]

The sulphonamide prepared above (5.78 mmol) and tin (II) chloride dihydrate (29 mmol) were added to ethyl acetate (40 ml). The reaction mixture was stirred for 1.5 hours at 60°C and then poured into ice and basified with an aqueous solution of sodium hydroxide. A precipitate formed which was filtered off and the product extracted from the aqueous phase with ethyl acetate. This extract was dried (MgSO₄) and the solvent evaporated to give di-[N-ethyl]-4,5-dimethoxy-2-aminobenzenesulphonamide which was used without further purification.

The aniline prepared above (5.78mmol) was stirred in acetic anhydride (20 ml) at 0°C. After 2 hours the solution was poured into ice and the product extracted with ethyl acetate. This extract was washed with a saturated aqueous solution of sodium hydrogen carbonate, dried (MgSO₄) and the solvent evaporated to give 4,5-methoxy-2-acetylaminodi-[N-ethyl]benzenesulphonamide (84%). NMR (DMSO-d₆): 1.01(t,6H); 2.09(s,3H); 3.19(m,4H); 3.80(s,6H); 7.25(s,1H); 7.60(s,1H); 9.26(br s,1H): M/S CI NH₃ (M+H)⁺=331.

Boron tribromide (2.22 mmol) was added to a solution of the acylated aniline prepared above (0.54 mmol) in dichloromethane (3 ml). The reaction mixture was stirred for 4 hours before being quenched by the addition of crushed ice. A 2N aqueous solution of sodium hydroxide was added and the organic and aqueous phases separated. The aqueous phase was acidified with a 2N aqueous solution of hydrochloric acid and extracted with ethyl acetate. This was dried (MgSO₄) and evaporated to give 4,5-dihydroxy-2-acetylamino di-[N-ethyl]benzenesulphonamide (95%).
NMR (DMSO-d₆): 1.00(t,6H); 2.05(s,3H); 3.15(m,4H); 7.14(s,1H); 7.44(s,1H); 9.06(br s,1H); 9.39(br s,1H); 10.00(br s,1H): M/S CI NH₃ (M+H)⁺=303.

Potassium carbonate (2.6 mmol) was added to a solution of the diol prepared above (0.52 mmol) in DMF (2 ml). Allyl bromide (2 mmol) in DMF (1 ml) was added dropwise to the reaction mixture and stirred for 5 hours. The mixture was left for 20 hours unstirred and filtered to remove the potassium carbonate. The filtrate was partitioned between ethyl acetate and water. The organic phase was washed with brine, an aqueous solution of sodium hydrogen carbonate and brine again. The organic phase was dried (MgSO₄) and the solvent evaporated to give 4,5-diallyloxy-2-acetylaminodi-[N-ethyl]benzenesulphonamide (80%).
NMR (DMSO-d₆): 1.00(t,6H); 2.09(s,3H); 3.19(m,4H); 4.60(m,4H); 5.22-5.55(m,4H); 5.92-6.16(m,2H); 7.24(s,1H); 7.65(s,1H); 9.25(s,1H): M/S CI NH₃ (M+H)⁺=383.

A mixture of the diallyloxybenzenesulphonamide compound from the above reaction (0.42 mmol), concentrated hydrochloric acid (0.5 ml), water (4.4 ml) and methanol (3 ml) were heated at reflux for 3 hours. The solvents were evaporated and the residue dried under a high vacuum to give 4,5-diallyloxy-2-aminodi-[N-ethyl]benzenesulphonamide (88%).
NMR (DMSO-d₆): 1.01(t,6H); 3.15(m,4H); 4.15(br, partially obscured, 2H); 4.42(m,2H); 5.18-5.50(m,4H); 5.89-6.15(m,2H); 6.48(s,1H); 6.95(s,1H): M/S CI NH₃ (M+H)⁺=341.

## Claims

1. A carbapenem compound of the formula (I): 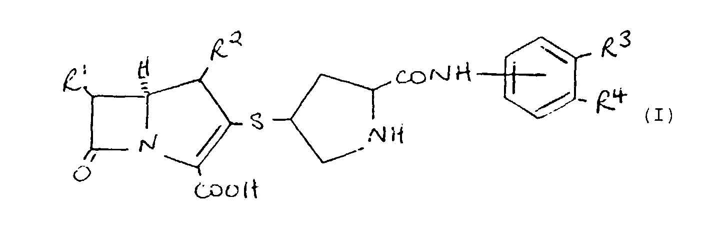 wherein:
R¹ is 1-hydroxyethyl, 1-fluoroethyl or hydroxymethyl;
R² is hydrogen or C₁₋₄alkyl;
R³ and R⁴ are ortho with respect to one another wherein R³ and R⁴ are independently hydroxy or in vivo hydrolysable esters thereof;
the benzene ring being optionally further substituted by C₁₋₄alkyl, halo, hydroxy, hydroxyC₁₋₄alkyl, amino, nitro, C₁₋₄alkoxy, carboxyC₁₋₄alkyl, C₁₋₄alkanoylamino, N-alkyl-N-C₁₋₄alkanoylamino, trifluoromethyl, carboxy, carbamoyl, C₁₋₄alkylcarbamoyl, di-C₁₋₄alkylcarbamoyl, cyano, C₁₋₄alkanesulphonamido, C₁₋₄alkanoyl, C₁₋₄alkanoyloxy, C₁₋₄alkoxycarbonyl, C₁₋₄alkylthio, C₁₋₄alkanesulphinyl, C₁₋₄alkanesulphonyl, C₂₋₄alkenyl, hydroxyiminomethyl (HON=CH-), C₁₋₄alkoxyiminomethyl, aminosulphonyl, N-C₁₋₄alkylaminosulphonyl or di-[N-C₁₋₄alkyl]aminosulphonyl;
or a pharmaceutically acceptable salt or in vivo hydrolysable ester thereof.

2. A compound according to claim 1 wherein R¹ is 1-hydroxyethyl.

3. A compound according to either claim 1 or claim 2 wherein R² is hydrogen.

4. A compound according to any one of the claims 1 to 3 wherein the benzene ring is optionally further substituted by fluoro, bromo, chloro, cyano, nitro, carboxymethyl, hydroxy, di-[N-methyl]carbamoyl, methanesulphonyl, di-[N-ethyl]aminosulphonyl or methoxycarbonyl.

5. A compound according to any one of claims 1 to 4, of the formula (IV): 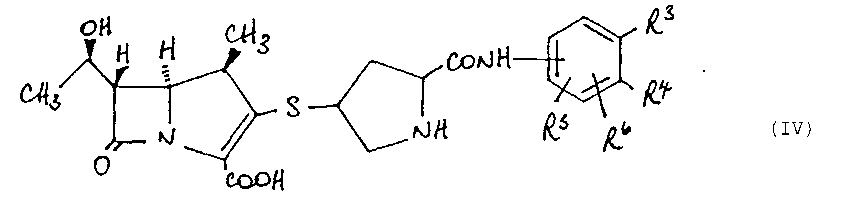 wherein R³ and R⁴ are as defined in claim 1 and R⁵ and R⁶ are independently hydrogen, halo, cyano, nitro, carboxy, carboxymethyl, methanesulphonyl, di-[N-methyl]carbamoyl, di-[N-ethyl]aminosulphonyl and hydroxy.

6. A compound according to any one of claims 1 to 4 wherein the benzene ring is substituted by R³ and R⁴ in positions 3 and 4 relative to the amido linking group.

7. A compound according to claim 1 which is
(5R,6S,8R,2'S,4'S)-2-(2-(3,4-dihydroxyphenylcarbamoyl)pyrrolidin-4-ylthio)-6-(1-hydroxyethyl)carbapenem-3-carboxylic acid;
(1R,5R,6S,8R,2'S,4'S)-2-(2-(5-cyano-3,4-dihydroxyphenylcarbamoyl)pyrrolidin-4-ylthio)-6-(1-hydroxyethyl)-1-methylcarbapenem-3-carboxylic acid;
(5R,6S,8R,2'S,4'S)-2-(2-(5-bromo-3,4-dihydroxyphenylcarbamoyl)pyrrolidin-4-ylthio)-6-(1-hydroxyethyl)carbapenem-3-carboxylic acid;
(1R,5R,6S,8R,2'S,4'S)-2-(2-(3,4-dihydroxyphenylcarbamoyl)pyrrolidin-4-ylthio)-6-(1-hydroxyethyl)-1-methylcarbapenem-3-carboxylic acid;
(1R,5R,6S,8R,2'S,4'S)-2-(2-(5-methoxy-3,4-dihydroxyphenylcarbamoyl)pyrrolidin-4-ylthio)-6-(1-hydroxyethyl)-1-methylcarbapenem-3-carboxylic acid;
(1R,5R,6S,8R,2'S,4'S)-2-(2-(2,5-dichloro-3,4-dihydroxyphenylcarbamoyl)pyrrolidin-4-ylthio)-6-(1-hydroxyethyI)-1-methylcarbapenem-3-carboxylic acid;
(1R,5R,6S,8R,2'S,4'S)-2-(2-(5-hydroxy-3,4-dihydroxyphenylcarbamoyl)pyrrolidin-4-ylthio)-6-(1-hydroxyethyl)-1-methylcarbapenem-3-carboxylic acid;
(1R,5R,6S,8R,2'S,4'S)-2-(2-(5-bromo-3,4-dihydroxyphenylcarbamoyl)pyrrolidin-4-ylthio)-6-(1-hydroxyethyl)-1-methylcarbapenem-3-carboxylic acid;
(1R,5R,6S,8R,2'S,4'S)-2-(2-(5-N-methoxyiminomethyl-3,4-dihydroxyphenylcarbamoyl)pyrrolidin-4-ylthio)-6-(1-hydroxyethyl)-1-methylcarbapenem-3-carboxylic acid;
(1R,5R,6S,8R,2'S,4'S)-2-(2-(6-methoxycarbonyl-3,4-dihydroxyphenylcarbamoyl)pyrrolidin-4-ylthio)-6-(1-hydroxyethyI)-1-methylcarbapenem-3-carboxylic acid;
(1R,5R,6S,8R,2'S,4'S)-2-(2-(6-carboxy-3,4-dihydroxyphenylcarbamoyl)pyrrolidin-4-ylthio)-6-(1-hydroxyethyl)-1-methylcarbapenem-3-carboxylic acid;
(1R,5R,6S,8R,2'S,4'S)-2-(2-(6-di-(N-ethyl)aminosulphonyl-3,4-dihydroxphenylcarbamoyl)pyrrolidin-4-ylthio)-6-(1-hydroxyethyl)-1-methylcarbapenem-3-carboxylic acid;
or a pharmaceutically acceptable salt or in vivo hydrolysable ester thereof.

8. A pharmaceutical composition which comprises a compound according to any one of claims 1 to 7 and a pharmaceutically acceptable carrier.

9. A process for preparing a compound according to claim 1 which comprises:
deprotecting a compound of the formula (V): 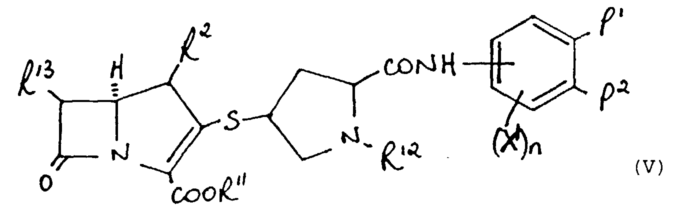 wherein R² is as defined in claim 1; R¹³ is a group R¹ as defined in claim 1 or 1-(protected hydroxy)ethyl; R¹¹ is hydrogen or a carboxy protecting group; R¹² is hydrogen or an amino protecting group, P¹ and P² are independently hydroxy or protected hydroxy and (X¹)ₙ represents up to three optional substituents which may be the same or different selected from C₁₋₄alkyl, halo, hydroxy, hydroxyC₁₋₄alkyl, amino, nitro, C₁₋₄alkoxy, carboxyC₁₋₄alkyl, C₁₋₄alkanoylamino,
N-alkyl-N-C₁₋₄alkanoylamino, trifluoromethyl, carboxy, carbamoyl, C₁₋₄alkylcarbamoyl, di-C₁₋₄alkylcarbamoyl, cyano, C₁₋₄alkanesulphonamido, C₁₋₄alkanoyl, C₁₋₄alkanoyloxy, C₁₋₄alkoxycarbonyl, C₁₋₄alkylthio, C₁₋₄alkanesulphinyl, C₁₋₄alkanesulphonyl, C₂₋₄alkenyl, hydroxyiminomethyl (HON=CH-), C₁₋₄alkoxyiminomethyl, aminosulphonyl, N-C₁₋₄alkylaminosulphonyl and di-[N-C₁₋₄alkyl]aminosulphonyl; with the proviso that there is at least one protecting group:
and thereafter if necessary:
(i) forming a pharmaceutically acceptable salt,
(ii) esterifying to form an in vivo hydrolysable ester.

10. A process for preparing a compound of the formula (I), as defined in claim 1, and a compound of the formula (V), as defined in claim 9;
or a pharmaceutically acceptable salt or in vivo hydrolysable ester thereof;
which comprises:
a) reacting compounds of the formulae (VI) and (VII): 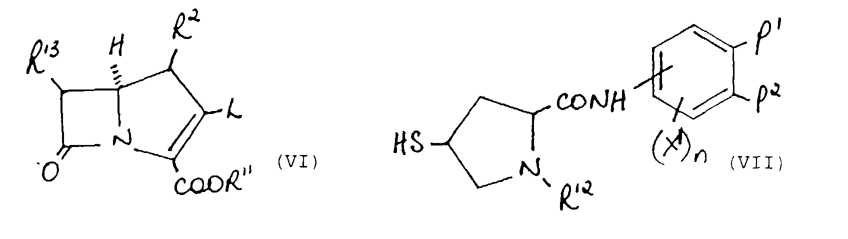 wherein P¹, P², R², X¹, n, R¹¹, R¹² and R¹³ are as defined in claim 9 and L is a leaving group, or
b) cyclising a compound of the formula (VIII): 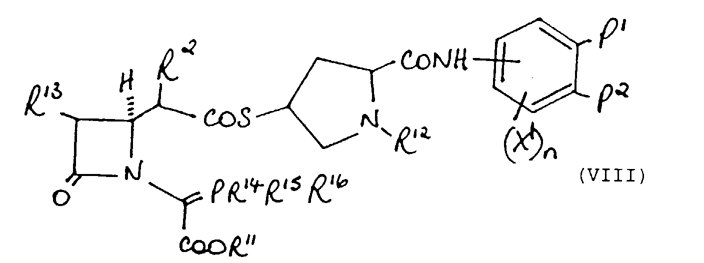 wherein P¹, P², X¹, n, R², R¹¹, R¹² and R¹³ are as defined in claim 9 and R¹⁴, R¹⁵ and R¹⁶ are independently selected from C₁₋₆alkoxy, aryloxy, di-C₁₋₆alkylamino and diarylamino or any two of R¹⁴⁻R¹⁶ represent o-phenylenedioxy; wherein any functional group is optionally protected and thereinafter if necessary:
(i) removing any protecting groups;
(ii) forming a pharmaceutically acceptable salt;
(iii) esterifying to form an in vivo hydrolysable ester.

11. A compound of the formula (V) as defined in claim 9.

12. A compound of the formula (VII) or (VIII) as defined in claim 10.

13. A compound of the formula (IX), (XII) or (XIV): 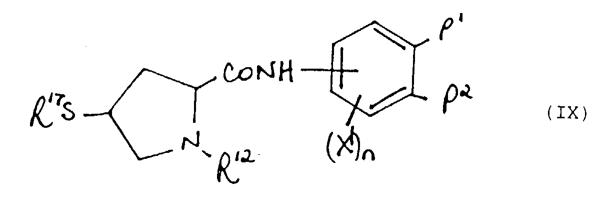 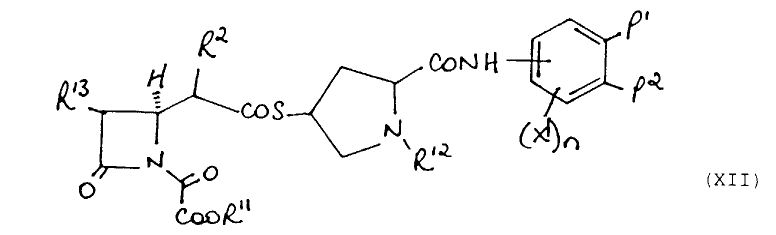 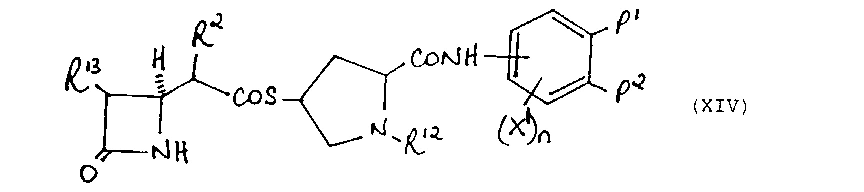 wherein R², R¹¹, R¹², R¹³, P¹, P² and (X¹)ₙ are as defined in claim 9 and R¹⁷ is a protecting group.

## Patentansprüche

1. Carbapenemverbindung der Formel (I): 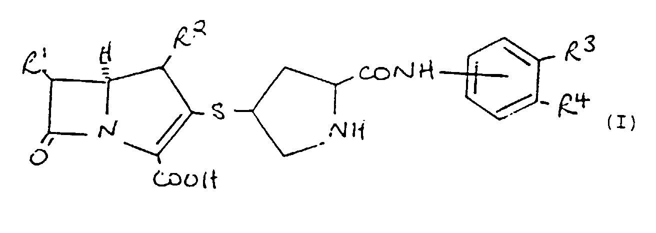 in der
R¹ eine 1-Hydroxyethyl-, 1-Fluorethyl- oder Hydroxymethylgruppe bedeutet,
R² ein Wasserstoffatom oder einen C₁₋₄-Alkylrest darstellt,
R³ und R⁴ zueinander ortho-ständig sind, wobei R³ und R⁴ unabhängig von einander eine Hydroxygruppe oder in vivo hydrolysierbare Estergruppen davon bedeuten,
der Benzolring gegebenenfalls weiter durch einen C₁₋₄-Alkylrest, ein Halogenatom, eine Hydroxygruppe, einen Hydroxy-C₁₋₄-alkylrest, eine Aminogruppe, eine Nitrogruppe, einen C₁₋₄-Alkoxyrest, einen Carboxy-C₁₋₄-alkylrest, einen C₁₋₄-Alkanoylaminorest, einen N-Alkyl-N-C₁₋₄-alkanoylaminorest, eine Trifluormethylgruppe, eine Carboxygruppe, eine Carbamoylgruppe, einen C₁₋₄-Alkylcarbamoylrest, einen Di-C₁₋₄-alkylcarbamoylrest, eine Cyanogruppe, einen C₁₋₄-Alkansulfonamidorest, einen C₁₋₄-Alkanoylrest, einen C₁₋₄-Alkanoyloxyrest, einen C₁₋₄-Alkoxycarbonylrest, einen C₁₋₄-Mkyllhiorest, einen C₁₋₄-Alkansulfinylrest, einen C₁₋₄-Alkansulfonylrest, einen C₂₋₄-Alkenylrest, eine Hydroxyiminomethylgruppe (HON=CH-), einen C₁₋₄-Alkoxyiminomethylrest, eine Aminosulfonylgruppe, einen N-C₁₋₄-Alkylaminosulfonylrest oder einen Di-[N-C₁₋₄-alkyl]aminosulfonylrest substituiert ist,
oder ein pharmazeutisch verträgliches Salz oder in vivo hydrolysierbarer Ester davon.

2. Verbindung nach Anspruch 1, wobei R¹ eine 1-Hydroxyethylgruppe bedeutet.

3. Verbindung nach Anspruch 1 oder Anspruch 2, wobei R² ein Wasserstoffatom darstellt.

4. Verbindung nach einem der Ansprüche 1 bis 3, wobei der Benzolring gegebenenfalls weiter durch ein Fluor-, Brom-, Chloratom, eine Cyano-, Nitro-, Carboxymethyl-, Hydroxy-, Di-[N-methyl]carbamoyl-, Methansulfonyl-, Di-[N-ethyl]aminosulfonyl- oder Methoxycarbonylgruppe substituiert ist.

5. Verbindung nach einem der Ansprüche 1 bis 4 der Formel (IV): 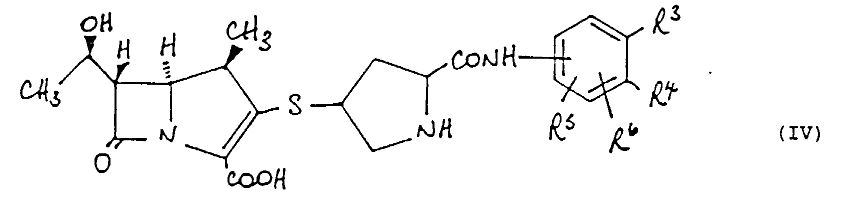 in der R³ und R⁴ wie in Anspruch 1 definiert sind und R⁵ und R⁶ unabhängig voneinander ein Wasserstoffatom, ein Halogenatom, eine Cyano-, Nitro-, Carboxy-, Carboxymethyl-, Methansulfonyl-, Di-[N-methyl]carbamoyl-, Di-[N-ethyl]aminosulfonyl- und Hydroxygruppe bedeuten.

6. Verbindung nach einem der Ansprüche 1 bis 4, wobei der Benzolring in den Stellungen 3 und 4 bezüglich der Amidbindungsgruppe mit R³ und R⁴ substituiert ist.

7. Verbindung nach Anspruch 1, nämlich
(5R,6S,8R,2'S,4'S)-2-(2-(3,4-Dihydroxyphenylcarbamoyl)pyrrolidin-4-ylthio)-6-(1-hydroxyethyl)carbapenem-3-carbonsäure;
(1R,5R,6S,8R,2'S,4'S)-2-(2-(5-Cyano-3,4-dihydroxyphenylcarbamoyl)pyrrolidin-4-ylthio)-6-(1-hydroxyethyl)-1-methylcarbapenem-3-carbonsäure;
(5R,65,8R,2'S,4'S)-2-(2-(5-Brom-3,4-dihydroxyphenylcarbamoyl)pyrrolidin-4-ylthio)-6-(1-hydroxyethyl)carbapenem-3 -carbonsäure;
(1R,5R,6S,8R,2'S,4'S)-2-(2-(3,4-Dihydroxyphenylcarbamoyl)pyrrolidin-4-ylthio)-6-(1-hydroxyethyl)-1-methylcarbapenem-3-carbonsäure;
(1R,5R,6S,8R,2'S,4'S)-2-(2-(5-Methoxy-3,4-dihydroxyphenylcarbamoyl)pyrrolidin-4-ylthio)-6-(1-hydroxyethyl)-1-methylcarbapenem-3-carbonsäure;
(1R,5R,6S,8R,2'S,4'S)-2-(2-(2,5-Dichlor-3,4-dihydroxyphenylcarbamoyl)pyrrolidin-4-ylthio)-6-(1-hydroxyethyl)-1 -methylcarbapenem-3-carbonsäure;
(1R,5R,6S,8R,2'S,4'S)-2-(2-(3,4,5-Trihydroxyphenylcarbamoyl)pyrrolidin-4-ylthio)-6-(1-hydroxyethyl)-1-methylcarbapenem-3-carbonsäure;
(1R,5R,6S,8R,2'S,4'S)-2-(2-(5-Brom-3,4-dihydroxyphenylcarbamoyl)pyrrolidin-4-ylthio)-6-(1-hydroxyethyl)-1-methylcarbapenem-3-carbonsäure;
(1R,5R,6S,8R,2'S,4'S)-2-(2-(5-N-Methoxyiminomethyl-3,4-dihydroxyphenylcarbamoyl)pyrrolidin-4-ylthio)-6-(1-hydroxyethyl)-1-methylcarbapenem-3-carbonsäure;
(1R,5R,6S,8R,2'S,4'S)-2-(2-(6-Methoxycarbonyl-3,4-dihydroxyphenylcarbamoyl)pyrrolidin-4-ylthio)-6-(1-hydroxyethyl)-1-methylcarbapenem-3-carbonsäure;
(1R,5R,6S,8R,2'S,4'S)-2-(2-(6-Carboxy-3,4-dihydroxyphenylcarbamoyl)pyrrolidin-4-ylthio)-6-(1-hydroxyethyl)-1-methylcarbapenem-3-carbonsäure;
(1R,5R,6S,8R,2'S,4'S)-2-(2-(6-Di-(N-ethyl)aminosulfonyl-3,4-dihydroxyphenylcarbamoyl)pyrrolidin-4-ylthio)-6-(1-hydroxyethyl)-1-methylcarbapenem-3-carbonsäure; oder ein pharmazeutisch verträgliches Salz oder in vivo hydrolysierbarer Ester davon.

8. Arzneimittel, das eine Verbindung nach einem der Ansprüche 1 bis 7 und einen pharmazeutisch verträglichen Träger umfaßt.

9. Verfahren zum Herstellen einer Verbindung nach Anspruch 1, das: die Schutzgruppenabspaltung aus einer Verbindung der Formel (V): 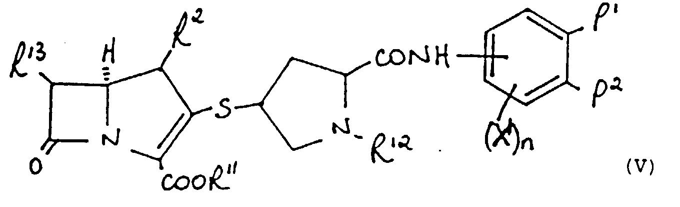 in der R² wie in Anspruch 1 definiert ist, R¹³ einen wie in Anspruch 1 definierten Rest R¹ oder eine geschützte 1-Hydroxyethylgruppe darstellt, R¹¹ ein Wasserstoffatom oder eine Carboxyschutzgruppe bedeutet, R¹² ein Wasserstoffatom oder eine Aminoschutzgruppe darstellt, P¹ und P² unabhängig voneinander eine Hydroxy- oder geschützte Hydroxygruppe bedeuten und (X¹)ₙ bis zu drei mögliche Substituenten darstellt, die gleich oder verschieden sein können und aus einem C₁₋₄-Alkylrest, einem Halogenatom, einer Hydroxygruppe, einem Hydroxy-C₁₋₄-alkylrest, einer Aminogruppe, einer Nitrogruppe, einem C₁₋₄-Alkoxyrest, einem Carboxy-C₁₋₄-alkylrest, einem C₁₋₄-Alkanoylaminorest, einem N-Alkyl-N-C₁₋₄-alkanoylaminorest, einer Trifluormethylgruppe, einer Carboxygruppe, einer Carbamoylgruppe, einem C₁₋₄-Alkylcarbamoylrest, einem Di-C₁₋₄-alkylcarbamoylrest, einer Cyanogruppe, einem C₁₋₄-Alkansulfonamidorest, einem C₁₋₄-Alkanoylrest, einem C₁₋₄-Alkanoyloxyrest, einem C₁₋₄-Alkoxycarbonylrest, einem C₁₋₄-Alkylthiorest, einem C₁₋₄-Alkansulfinylrest, einem C₁₋₄-Alkansulfonylrest, einem C₂₋₄-Alkenylrest, einer Hydroxyiminomethylgruppe (HON=CH-), einem C₁₋₄-Alkoxyiminomethylrest, einer Aminosulfonylgruppe, einem N-C₁₋₄-Alkylaminosulfonylrest und einem Di-[N-C₁₋₄-alkyl]aminosulfonylrest ausgewählt werden; mit der Maßgabe, daß es mindestens eine Schutzgruppe gibt:
und danach, falls notwendig:
(i) Herstellung eines pharmazeutisch verträglichen Salzes,
(ii) Veresterung unter Herstellung eines in vivo hydrolysierbaren Esters umfaßt.

10. Verfahren zum Herstellen einer wie in Anspruch 1 definierten Verbindung der Formel (I) und einer wie in Anspruch 9 definierten Verbindung der Formel (V) oder eines pharmazeutisch verträglichen Salzes oder in vivo hydrolysierbaren Esters davon, das:
(a) Umsetzung von Verbindungen der Formeln (VI) und (VII): 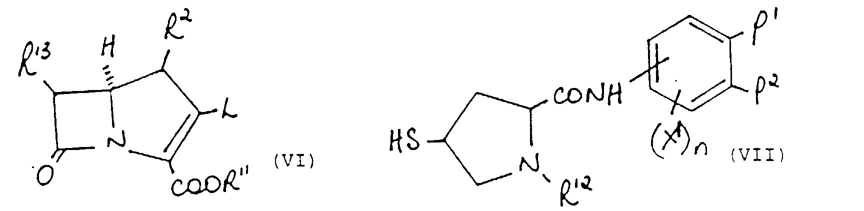 in denen P¹, P², R², X¹, n, R¹¹, R¹² und R¹³ wie in Anspruch 9 definiert sind und L eine Abgangsgruppe darstellt, oder
(b) Cyclisierung einer Verbindung der Formel (VIII): 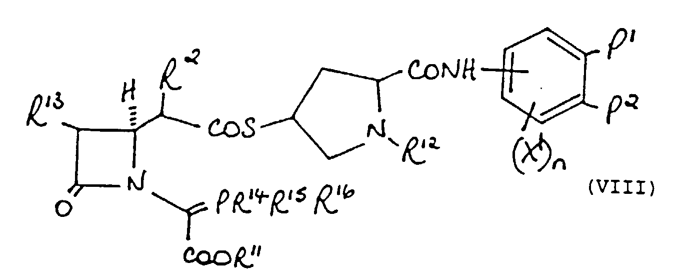 in der P¹, P², X¹, n, R², R¹¹, R¹² und R¹³ wie in Anspruch 9 definiert sind und R¹⁴, R¹⁵ und R¹⁶ unabhängig voneinander aus einem C₁₋₆-Alkoxy-, Aryloxy-, Di-C₁₋₆-alkylamino- und Diarylaminorest ausgewählt werden oder zwei beliebige Reste R¹⁴ - R¹⁶ eine o-Phenylendioxygruppe darstellen, wobei eine beliebige funktionelle Gruppe gegebenenfalls geschützt ist, und danach, falls notwendig:
(i) Entfernung irgendwelcher Schutzgruppen;
(ii) Herstellung eines pharmazeutisch verträglichen Salzes;
(iii) Veresterung unter Herstellung eines in vivo hydrolysierbaren Esters umfaßt.

11. Verbindung der Formel (V), wie in Anspruch 9 definiert.

12. Verbindung der Formel (VII) oder (VIII), wie in Anspruch 10 definiert.

13. Verbindung der Formel (IX), (XII) oder (XIV): 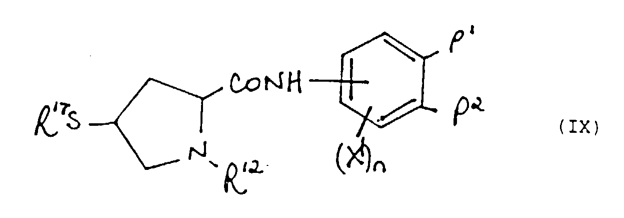 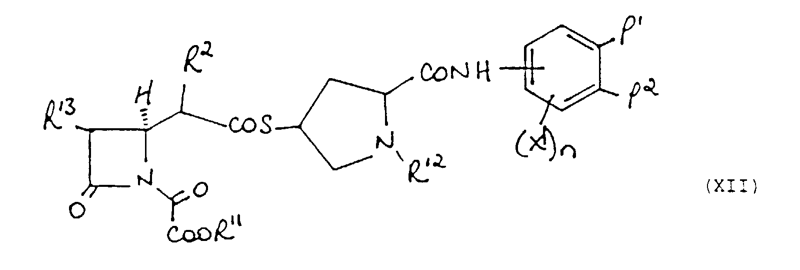 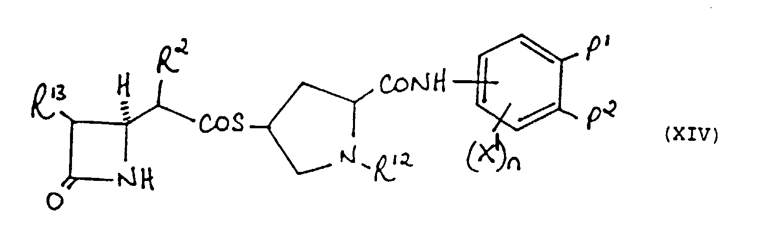 in denen R², R¹¹, R¹², R¹³, P¹, P² und (X¹)ₙ wie in Anspruch 9 definiert sind und R¹⁷ eine Schutzgruppe darstellt.

## Revendications

1. Composé carbapénème de formule (I) 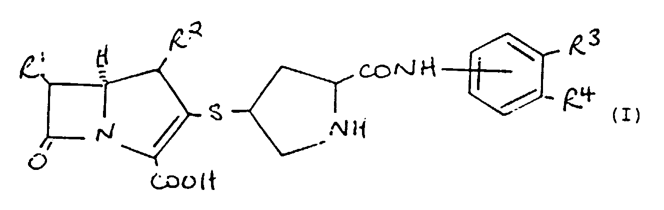 dans laquelle :
- R¹ représente un groupe 1-hydroxyéthyle, 1-fluoroéthyle, ou hydroxyméthyle ;
- R² représente un atome d'hydrogène ou un groupe alkyle C₁₋₄ ;
- R³ et R⁴ sont en position ortho l'un par rapport à l'autre, et représentent indépendamment, un groupe hydroxy ou ester de celui-ci hydrolysable in vivo; le cycle benzénique étant éventuellement encore substitué par un groupe alkyle C₁₋₄, halo, hydroxy, hydroxyalkyle C₁₋₄, amino, nitro, alcoxy C₁₋₄, carboxyalkyle C₁₋₄, alcanoyl C₁₋₄ amino, N-alkyl-N-alcanoyl C₁₋₄ amino, trifluorométhyle, carboxy, carbamoyle, alkyl C₁₋₄ carbamoyle, dialkyl C₁₋₄ carbamoyle, cyano, alcane C₁₋₄ sulfonamido, alcanoyle C₁₋₄, alcanoyl C₁₋₄ oxy, alcoxy C₁₋₄ carbonyle, alkyl C₁₋₄ thio, alcane C₁₋₄ sulfinyle, alcane C₁₋₄ sulfonyle, alkényle C₂₋₄, hydroxyiminoéthyle (HON=CH-), alcoxy C₁₋₄ iminométhyle, aminosulfonyle, N-alkyl C₁₋₄ aminosulfonyle, ou di-(N-alkyl C₁₋₄)-aminosulfonyle
ou un sel pharmaceutiquement acceptable ou un ester hydrolysable in vivo, de celui-ci.

2. Composé selon la revendication 1, dans lequel R¹ est un groupe 1-hydroxyéthyle.

3. Composé selon la revendication 1, ou la revendication 2, dans lequel R² est un atome d'hydrogène.

4. Composé selon l'une quelconque des revendications 1 à 3, dans lequel le cycle benzénique est éventuellement substitué aussi par des groupes fluoro, bromo, chlore, cyano, nitro, carboxyméthyle, hydroxy, di-(N-méthyl)carbamoyle, méthanesulfonyle, di-(N-éthyl)aminosulfonyle, ou méthoxycarbonyle.

5. Composé selon l'une quelconque des revendications 4, de formule (IV): 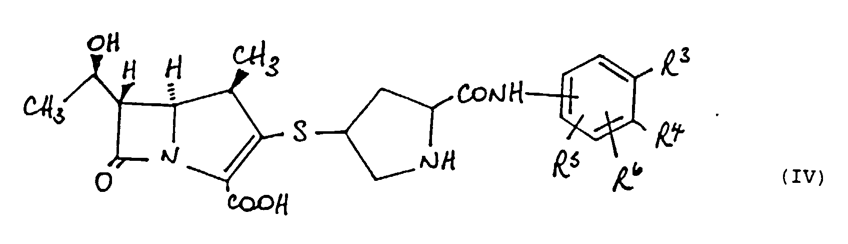 dans laquelle R³ et R⁴ sont tels que définis dans la revendication 1, et R⁵ et R⁶ représente indépendamment, un atome d'hydrogène, un groupe halo, cyano, nitro, carboxy, carboxyméthyle, méthanesulfonyle, di-(N-méthyl)carbamoyle, di-(N-éthyl)aminosulfonyle, ou hydroxy.

6. Composé selon l'une quelconque des revendications 1 à 4, dans lequel le cycle benzénique est substitué par R³ et R⁴ en position 3 et en position 4 par rapport au groupe de liaison amido.

7. Composé selon la revendication 1, qui est :
- l'acide (5R, 6S, 8R, 2'S, 4'S)-2-[2-(3,4-dihydroxyphénylcarbamoyl)pyrrolidin-4-ylthio]-6-(1-hydroxyéthyl )carbapénème-3-carboxylique ;
- l'acide (1R, 5R, 6S, 8R, 2'S, 4'S)-2-[2-(5-cyano-3,4-dihydroxyphénylylcarbamoyl)pyrrolid in-4-ylthio]-6-(1-hydroxyéthyl)-1-méthylcarbapénème-3-carboxylique ;
- l'acide (5R, 6S, 8R, 2'S, 4'S)-2-[2-(5-bromo-3,4-dihydroxyphénylcarbamoyl)pyrrolidin-4-ylthio]-6-(1-hydroxyéthyl)carbapénème-3-carboxylique ;
- l'acide (1R, 5R, 6S, 8R, 2'S, 4'S)-2-[2-(3,4-dihydroxyphénylcarbamoyl)pyrrolidin-4-ylthio]-6-(1-hydroxyéthyl)-1-méthylcarbapènéme-3carboxylique ;
- l'acide (1R, 5R, 6S, 8R, 2'S, 4'S)-2-[2-(5-méthoxy-3,4-dihydroxyphénylcarbamoyl)pyrrolidin-4-ylthio]-6-(1-hydroxyéthyl)-1-méthylcarbapénème-3-carboxylique ;
- l'acide (1R, 5R, 6S, 8R, 2'S, 4'S)-2-[2-(2,5-dichloro-3,4-dihydroxyphénylcarbamoyl)pyrrolidin-4-ylthio]-6-(1-hydroxyéthyl)-1-méthylcarbapénème-3-carboxylique ;
- l'acide (1R, 5R, 6S, 8R, 2'S, 4'S)-2-[2-(5-hydroxy-3,4-dihydroxyphénylcarbamoyl)pyrrolidin-4-ylthio]-6-(1-hydroxyéthyl)-1-méthylcarbapénème-3-carboxylique ;
- l'acide (1R, 5R, 6S, 8R, 2'S, 4'S)-2-[2-(5-bromo-3,4-dihydroxyphénylcarbamoyl)pyrrolidin-4-ylthio]-6-(1-hydroxyéthyl)-1-méthylcarbapénème-3-carboxylique ;
- l'acide (1R, 5R, 6S, 8R, 2'S, 4'S)-2-[2-(5-N-méthoxyiminométhyl-3,4-dihydroxyphénylcarbamoyl)pyrrolidin-4-ylthio]-6-(1-hydroxyéthyl)-1-méthylcarbapénème-3-carboxylique ;
- l'acide (1R, 5R, 6S, 8R, 2'S, 4'S)-2-[2-(6-méthoxycarbonyl-3,4-dihydroxyphénylcarbamoyl)pyrrolidin-4-ylthio]-6-(1-hydroxyéthyl)-1-méthylcarbapénème-3-carboxylique ;
- l'acide (1R, 5R, 65, 8R, 2'S, 4'S)-2-[2-(6-carboxy-3,4-dihydroxyphénylcarbamoyl)pyrrolidin-4-ylthio]-6-(1-hydroxyéthyl)-1-méthylcarbapénème-3-carboxylique ;
- l'acide (1R, 5R, 6S, 8R, 2'S, 4'S)-2-[2-(6-di-(N-éthyl)aminosulfonyl-3,4-dihydroxyphénylcarbamoyl)pyrrolidin-4-ylthio]-6-(1-hydroxyéthyl)-1-méthylcarbapénème-3-carboxylique ;
ou un sel pharmaceutiquement acceptable ou un ester hydrolysable in vivo, de ceux-ci.

8. Composition pharmaceutique qui comprend un composé selon l'une quelconque des revendications 1 à 7, et un véhicule pharmaceutiquement acceptable

9. Procédé de préparation d'un composé selon la revendication 1, qui comprend la déprotection d'un composé de formule (V): 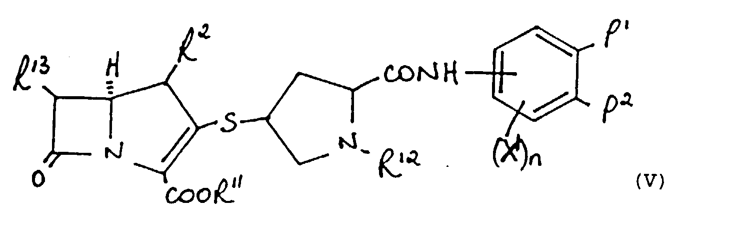 dans laquelle :
- R² est tel que défini dans la revendication 1 ;
- R¹³ est un groupe R¹ tel que défini dans la revendication 1 ou un groupe 1-(hydroxy protégé)éthyle ;
- R¹¹ est un atome d'hydrogène ou un groupe carboxy-protecteur ;
- R¹² est un atome d'hydrogène ou un groupe amino-protecteur ;
- P¹ et P² sont indépendamment, un groupe hydroxy ou hydroxy protégé ; et
- (X¹)ₙ représente jusqu'à trois substituants éventuels qui peuvent être identiques ou différents, et sont choisis parmi les groupes alkyle C₁₋₄, halo, hydroxy, hydroxyalkyle C₁₋₄, amino, nitro, alcoxy C₁₋₄, carboxyalkyle C₁₋₄, alcanoyl C₁₋₄ amino, N-alkyl-N-alcanoyl C₁₋₄ amino, trifluorométhyle, carboxy, carbamoyle, alkyl C₁₋₄ carbamoyle, dialkyl C₁₋₄ carbamoyle, cyano, alcane C₁₋₄ sulfonamido, alcanoyle C₁₋₄, alcanoyl C₁₋₄ oxy, alcoxy C₁₋₄ carbonyle, alkyl C₁₋₄ thio, alcane C₁₋₄ sulfinyle, alcane C₁₋₄ sulfonyle, alkényle C_{2-4,} hydroxyiminométhyle (HON=CH-), alcoxy C₁₋₄ iminométhyle, aminosulfonyle, N-alkyl C₁₋₄ aminosulfonyle, et di-(N-alkyl C₁₋₄)-aminosulfonyle ;
à condition qu'il y ait au moins un groupe protecteur ;
et, ensuite si nécessaire ;
- (i) la formation d'un sel pharmaceutiquement acceptable,
- (ii) l'estérification destinée à former un ester hydrolysable in vivo.

10. Procédé de préparation d'un composé de formule (I) selon la revendication 1, et d'un composé de formule (V) selon la revendication 9,
ou d'un sel pharmaceutiquement acceptable ou d'un ester hydrolysable in vivo, de celui-ci.
qui comprend :
(a) la réaction de composés de formules (VI) et (VII): 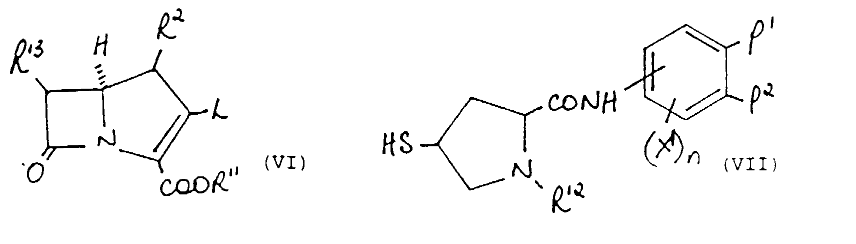 dans lesquelles P¹, P², R², X¹, n, R¹¹, R¹², et R¹³ sont tels que définis dans la revendication 9, et L est un groupe partant, ou
(b) la cyclisation d'un composé de formule (VIII) : 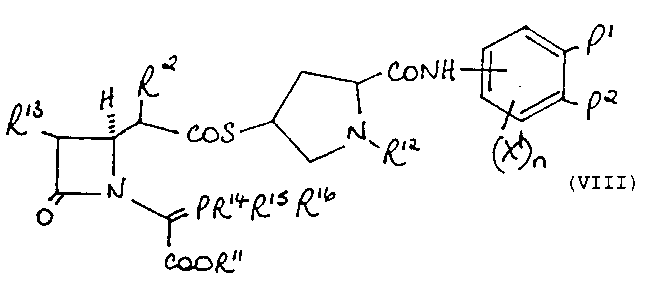 dans laquelle P¹, P², R², X¹, n, R¹¹, R¹², et R¹³ sont tels que définis dans la revendication 9, et R¹⁴, R¹⁵ et R¹⁶ sont choisis indépendamment parmi les groupes alcoxy C₁₋₆, aryloxy, dialkyl C₁₋₆ amino et diarylamino, ou bien deux quelconques de R¹⁴, R¹⁵ ou R¹⁶ représentent un groupe o-phénylènedioxy; et dans laquelle tout groupe fonctionnel est éventuellement protégé ; et ensuite si nécessaire :
- (i) le déplacement de tous les groupes protecteurs,
- (ii) la formation d'un sel pharmaceutiquement acceptable,
- (iii) l'estérification destinée à former un ester hydrolysable in vivo.

11. Composé de formule (V) selon la revendication 9.

12. Composé de formule (VII) ou de formule (VIII) selon la revendication 10.

13. Composé de formule (IX), de formule (XII) ou de formule (XIV) : 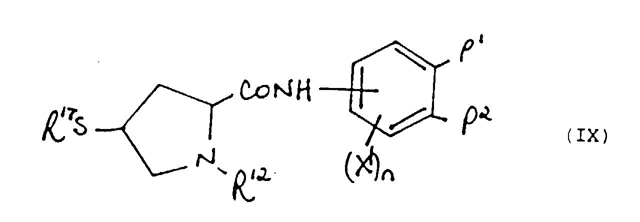 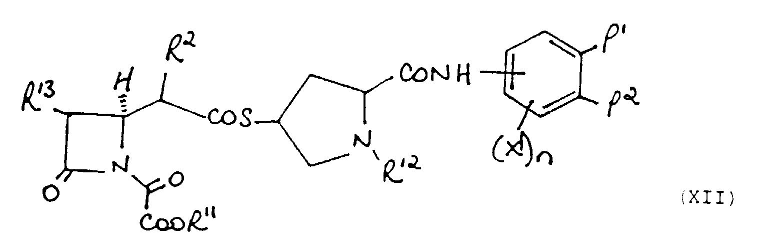 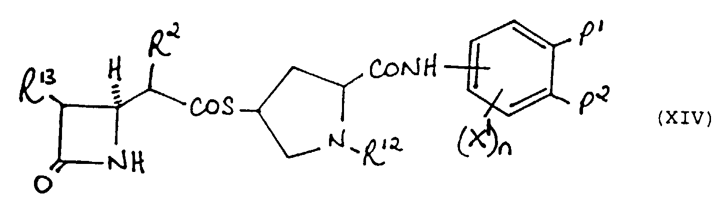 dans lesquelles R², R¹¹, R¹², R¹³ P¹, P² et (X¹)ₙ sont tels que définis dans la revendication 9, et R¹⁷ est un groupe protecteur.
